(19) Europäisches Patentamt / European Patent Office / Office européen des brevets

(11) **EP 3 760 181 B1**

(12) **EUROPÄISCHE PATENTSCHRIFT**

(45) Veröffentlichungstag und Bekanntmachung des Hinweises auf die Patenterteilung:
**26.10.2022   Patentblatt 2022/43**

(21) Anmeldenummer: **20189996.0**

(22) Anmeldetag: **24.01.2018**

(51) Internationale Patentklassifikation (IPC):
**A61K 6/00** *(2020.01)*     **A61K 6/30** *(2020.01)*
**A61K 6/71** *(2020.01)*     **A61K 6/887** *(2020.01)*

(52) Gemeinsame Patentklassifikation (CPC):
(C-Sets verfügbar)
**A61K 6/30; A61K 6/71; A61K 6/887**     (Forts.)

(54) **DENTALER KOMPOSITBLOCK ZUR HERSTELLUNG PERMANENTER INDIREKTER RESTAURATIONEN IM CAD/CAM VERFAHREN**

DENTAL COMPOSITE BLOCK FOR PREPARING PERMANENT INDIRECT RESTORATIONS IN CAD/CAM METHOD

BLOC COMPOSITE DENTAIRE DESTINÉ À LA FABRICATION DE RESTAURATIONS INDIRECTES PERMANENTES DANS LE PROCÉDÉ CAD/CAM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.02.2017   DE 102017103084**

(43) Veröffentlichungstag der Anmeldung:
**06.01.2021   Patentblatt 2021/01**

(62) Dokumentnummer(n) der früheren Anmeldung(en) nach Art. 76 EPÜ:
**18153169.0 / 3 363 423**

(73) Patentinhaber: VOCO GmbH
**27472 Cuxhaven (DE)**

(72) Erfinder:
• **Fontein, Nils**
  **27474 Cuxhaven (DE)**
• **Maletz, Reinhard**
  **27472 Cuxhaven (DE)**
• **Oldenburger, Daniel**
  **27476 Cuxhaven (DE)**
• **Plaumann, Manfred Thomas**
  **27472 Cuxhaven (DE)**

(56) Entgegenhaltungen:
**US-A1- 2014 200 284     US-A1- 2014 220 512**

• **SARA VANOORBEEK ET AL: "Computer-aided designed computer-assisted manufactured composite resin versus ceramic single-tooth restorations: A 3-year clinical study", THE INTERNATINAL JOURNAL OF PROSTHODONITICS, vol. 23, no. 3, 2010, pages 223-230,**
• **Schepke Ulf ET AL: "Clinical Bonding of Resin Nano Ceramic Restorations to Zirconia Abutments: A Case Series within a Randomized Clinical Trial : Clinical Bonding Failure of RNC Restorations to ZrO 2", CLINICAL IMPLANT DENTISTRY AND RELATED RESEARCH, vol. 18, no. 5, 12 October 2015 (2015-10-12), pages 984-992, XP055934548, CA ISSN: 1523-0899, DOI: 10.1111/cid.12382**

EP 3 760 181 B1

(52) Gemeinsame Patentklassifikation (CPC): (Forts.)

C-Sets
**A61K 6/887, C08L 33/06**

**Beschreibung**

Die vorliegende Erfindung betrifft dentale Fräsrohlinge auf Kunststoffbasis, sogenannte Kompositblöcke, zur Herstellung von Dentalprothesen in einem CAD/CAM Verfahren. Prothetische Versorgungen bilden einen Ersatz für Zähne und umfassen beispielsweise Kronen und Brücken, Teilkronen, Inlays, Onlays oder Verblendungen.

Der technische Fortschritt computergesteuerter Maschinen brachte es mit sich, dass Fräsmaschinen entwickelt wurden, die in der Lage sind, in kürzester Zeit und mit minimalem Aufwand prothetische Versorgungen in nicht gekannter Genauigkeit herzustellen. Vor diesem Hintergrund entwickelte sich die sogenannte "digitale Zahnmedizin". Sie ist heutzutage in der Zahnmedizintechnik von überragender Bedeutung.

Gefräst wurden anfangs ausschließlich keramische oder metallische Materialien, doch im Zuge der immer besser der natürlichen Zahnhartsubstanz angepassten dentalen Kompositmaterialien, wurde auch diese Substanzklasse für eine Verwendung als Fräsrohling interessant.

Anders als Kompositmaterialien, die durch gezielte Formulierung von Harzmatrix und Füllstoffzusammensetzung auf die mannigfachen Anforderungen eines Dentalmaterials in dem feindlichen Milieu der Mundhöhle eingestellt werden können, können keramische Materialien zu hohe Härtegrade aufweisen und neigen aufgrund ihrer inherenten Sprödigkeit zur Bruchbildung. Metallische Materialien lassen sich ästhetisch schlecht einstellen und verursachen bei Patienten oft allergische Reaktionen.

Die vorliegende Erfindung betrifft einen Hochleistungskompositblock zur Herstellung permanenter indirekter Restaurationen im CAD/CAM Verfahren. Unter einem dentalen Kompositmaterial versteht der Fachmann eine radikalisch polymerisierbare oder radikalisch polymerisierte Zusammensetzung, die wenigstens eine radikalisch polymerisierbare flüssige oder eine radikalisch polymerisierte feste Harzphase, eine Feststoffphase, umfassend Füllstoffe unterschiedlichster Art und Menge, einen oder mehrere Polymerisationsinitiator(en) sowie gegebenenfalls übliche Additive wie Inhibitoren, Farbmittel, Stabilisatoren, etc. enthält. Das noch nicht radikalisch gehärtete Kompositmaterial kann entweder chemisch und/oder thermisch und/oder photochemisch mittels Strahlung radikalisch polymerisiert werden.

Dentale Kompositblöcke, beziehungsweise dentale Fräsrohlinge, sind aus dem Stand der Technik bekannt.

Die DE 699 22 413 T2 beschreibt einen schneidfähigen Fräsrohling, der ein Polymerharz und ein feinverteiltes Füllstoffmaterial mit einem maximalen Partikeldurchmesser von weniger als 50 Mikrometer enthält. Die Harzphase der in diesem Dokument untersuchten dentalen Zusammensetzungen umfasst das System Bis-GMA (2,2-Bis[4-(2-hydroxy-3-methacryloyloxypropoxy)phenyl]propan)/TEGDMA (Triethylenglykoldimethacrylat), die Füllstoffphase enthält oberflächensilanisierte Kieselsäure und Glas.

Die EP 3 050 533 A1 offenbart dentale Harzblöcke, die als Harzphase UDMA/ TEGDMA (Urethandimethacrylat/ Triethylenglykoldimethacrylat) 80/20, UDMA/ Bis-MEPP/ HDP (Urethandimethacrylat/ 2,2-Bis(4-methacryloxypolyethoxyphenyl)propan/ 2-Hydroxy-1,3-dimethacyloxypropan 60/20/20 und 50/30/20 sowie UDMA/ Bis-MEPP (Urethandimethacrylat/ 2,2-Bis(4-methacryloxypolyethoxyphenyl)propan 60/40 verwenden. Leider geht aus dem Dokument nicht hervor, welches Urethandimethacrylat eingesetzt wird. Laut einer älteren Auslegeschrift (DE 26 56 847) desselben Anmelders liegt der Ethoxylierungsgrad des Bis-MEPP im Bereich von 2,2 bis 6. Dies bedeutet, dass zur Herstellung des Bis-MEPP das Bisphenol A mit 2,2 bis 6 mol Ethylenoxid umgesetzt wird und dieses Zwischenprodukt schließlich mit 2 mol Methacrylsäure abgesättigt wird. Diese Harzzusammensetzungen werden mit anorganischen Füllstoffen zu radikalisch härtbaren Pasten in Gewichtsverhältnissen von Füllstoff zu Harz von 64:36 bis 70,8 : 29,2 vermischt und dann zu Harzblöcken mit Hilfe von BPO (Benzoylperoxid) thermisch polymerisiert.

Die DE 24 62 271 C2 beansprucht dentale Formkörper, enthaltend mindestens ein polymerisiertes Acrylat bzw. Methacrylat und einen silanisierten mikrofeinen anorganischen Füllstoff auf Siliciumdioxidbasis, die dadurch gekennzeichnet sind, dass sie als polymerisiertes Acrylat bzw. Methacrylat ein Polymerisat aus Bis-GMA oder einem anderen Derivat des Bisphenols A oder einem Reaktionsprodukt aus Hydroxyethylmethacrylaten und Diisocyanaten, gegebenenfalls zusammen mit Polymerisaten aus kurzkettigen Methacrylsäureestern und/oder aus difunktionellen Acryl- bez. Methacrylsäureestern und als anorganischen Füllstoff ausschließlich mikrofeines Siliciumdioxid mit einer Teilchengröße von 10 bis 400 nm und mit einer BET-Oberfläche von weniger als 200 m$^2$/g in einer Menge von 20 bis 80%, bezogen auf das Gewicht des Werkstoffs, enthalten. Die Menge der mikrofeinen Kieselsäure kann im Bereich zwischen 40 bis 75 Gewichtsprozent, bezogen auf den Formkörper, liegen.

Die EP 2 623 065 B1 offenbart Rohlinge für eine Dentalfräse, die auf hohe mechanische Eigenschaften wie Biegefestigkeit und Glanzbeständigkeit abzielen. Erreicht werden diese Eigenschaften durch einen Rohling für eine Dentalfräse, der aus einem gehärteten Produkt einer aushärtbaren Zusammensetzung gebildet wird und umfasst: (a) ein polymerisierbares Monomer; (b) einen sphärischen anorganischen Füllstoff, der eine durchschnittliche Primärteilchengröße von nicht weniger als 0,1 μm und weniger als 1 μm aufweist und (c) einen anorganischen Ultrafeinteilchenaggregatfüllstoff, bestehend aus Aggregaten von anorganischen ultrafeinen Teilchen, die eine durchschnittliche Primärteilchengröße von 2 bis 50 nm aufweisen. Als Harzmatrix wird hier eine Monomermischung aus Bis-GMA/TEGDMA/Bis-MEPP (2,2-Bis[4-(2-hydroxy-3-methacryloyloxypropoxy)phenyl]propan)/ (Triethylenglykoldimethacrylat)/2,2-Bis(4-methacryloxypolyethoxyphenyl)propan im Gewichtsverhältnis 20/30/50 verwendet.

Die WO 2011/087832 A1 ist auf thermisch gehärtete Kompositrohlinge gerichtet, die signifikant verbesserte mechanische und ästhetische Eigenschaften aufweisen sollen. Die radikalisch härtbaren Zusammensetzungen enthalten Initiatoren, die thermisch zerfallen. Da der Zerfall bei höheren Temperaturen erfolgt, wird verhindert, dass Monomere unter normalen Verarbeitungsbedingungen vorzeitig gelieren und durch Abbauprozesse den Rohling verfärben.

Die WO 2016/140950 A1 beschreibt Kompositmaterialien, die im gehärteten Zustand auch als dentale Fräsrohlinge verwendet werden können. Die Zusammensetzungen enthalten eine härtbare Harzkomponente, Keramikfasern und Füllstoff in Form von Nanoclustern. Die Polymerisate sollen hochästhetische Restaurationen ergeben und ausgezeichnete Polierbarkeiten sowie Polierretentionseigenschaften aufweisen. In einem Ausführungsbeispiel wird die Zusammensetzung einer Harzphase angegeben, deren Bestandteile Bis-GMA, TEGDMA, UDMA, BisEMA-6 (ethoxyliertes Bisphenol A dimethacrylat mit 6 Ethoxygruppen) und PEG600DM (Polyethylenglykoldimethacrylat mit einem Molekulargewicht der Polyethyleneinheiten von ca. 600 g/mol) in Gewichtsverhältnissen von 25/1,2/35/35/3,8 verwendet wird.

Die EP 2 881 077 A1 zielt auf Fräsrohlinge ab, die ausgezeichnete mechanische Eigenschaften, eine ausgezeichnete Abriebfestigkeit und einen ausgezeichneten Oberflächenglanz aufweisen sollen. Diese Eigenschaften sollen hier verfahrenstechnisch erreicht werden. Im Gegensatz zu den herkömmlichen Herstellmethoden, bei denen das Kompositmaterial durch homogenes Mischen und Kneten einer härtbaren Monomermischung mit einem Füllstoff zu einer Paste verarbeitet wird, die eine bestimmte Fließfähigkeit aufweisen muss, um in einer Form zu einem Fräsrohling ausgehärtet zu werden, schlägt diese Anmeldung vor, den anorganischen Füllstoff heißzupressen, um ihn zu aggregieren und ihn schließlich mit dem härtbaren Harz zu infiltrieren. Das Harz soll so die Zwischenräume der Primärpartikel füllen und kann dann gehärtet werden. Dieses Verfahren soll die Herstellung von Rohlingen gestatten, bei denen die Füllstoffteilchen so dicht nebeneinander liegen, wie es beim konventionellen Herstellverfahren nicht möglich ist und somit erlauben, Fräsrohlinge mit einem besonders hohen Füllstoffgehalt zur Verfügung zu stellen.

Die US 6,345,984 B2 betrifft Fräsrohlinge, wobei das Kompositmaterial einen partikulären Füllstoff in einem Größenbereich von ungefähr 0,1 bis 5,0 $\mu$m und kolloidales Silikat in einer Größe von 1 bis ungefähr 70 nm sowie eine Harzphase aus ethoxyliertem BPA (Bisphenol A)-Dimethacrylat enthält und der Ethoxylierungsgrad von 1 bis 20, vorzugsweise von 2 bis 7 Mol Ethylenoxid/ mol BPA reicht. Der Fräsrohling besteht insgesamt aus ca. 20 bis ca. 30 Gew.- % aus einer organischen Matrix und aus ca. 65 bis ca. 85 Gew.-% partikulärem Füllstoff, wobei die organische Matrix aus ca. 65 bis ca. 90 Gew.-% ethoxyliertem BPA-Dimethacrylat und aus ca. 10 bis ca. 30 Gew.-% eines Methacrylatoligomers, beispielsweise eines Polycarbonatdimethacrylatkondensationsproduktes, besteht. Unter Berücksichtigung der in der US 6,345,984 B2 zitierten US 4,544,359 sieht die typische Zusammensetzung eines Fräsrohlings dann folgendermaßen aus: partikulärer Füllstoff (0,1 bis 5 $\mu$m) 65 bis 79 Gew.-%, kolloidales Silikat (1 bis 70 nm) 1 bis 5 Gew.-% und organische Matrix 20 bis 30 Gew.-%.

Die US 6,186,790 zeigt fertige dentale Brückenelemente, wobei das Kompositmaterial faserverstärkt ist.

Die DE 198 23 530 B4 ist auf ein Dentalharzmaterial gerichtet, das zu einer Zahnprothese durch Fräsen geformt wird, das ein Acrylharzpolymer umfasst, das 20 bis 70 Gew.-% eines anorganischen Füllstoffs mit einer mittleren Teilchengröße von 10 bis 40 nm im Durchmesser enthält, 1 bis 40 Gew.-% eines Glaspulvers mit einer mittleren Teilchengröße von 0,1 - 5 $\mu$m im Durchmesser und 1-40 Gew.-% eines organisch-anorganischen Verbundfüllstoffes, der durch Mischen und Härten eines Gemisches aus einem ultrafeinen anorganischen Füllstoff mit einer mittleren Teilchengröße von 10 bis 40 nm im Durchmesser und einem Methacrylat- oder Acrylatmonomeren mit mindestens einer ungesättigten Doppelbindung und Pulverisieren des gehärteten Gemisches derart, dass es eine mittlere Teilchengröße von 5 bis 50 $\mu$m im Durchmesser aufweist, hergestellt ist, wobei das Acrylharzpolymer eine Kombination aus einem Methacryl- oder Acrylatmonomeren mit mindestens einer ungesättigten Doppelbindung und einem Wärmepolymerisationsinitiator umfasst. In Beispiel 5 wird die Kompositzusammensetzung eines Fräsrohlings beschrieben. Die Harzphase umfasst UDMA/Bis-MEPP in einem Gewichtsverhältnis von 5/20 und die Feststoffphase enthält 22 Gew.-% anorganischen Füllstoff (beispielsweise Aerosil mit einer mittleren Teilchengröße von 10 bis 40 nm), beispielsweise OX-50 (mit 40 nm Primärpartikelgröße), 23 Gew.-% Quarzglaspulver (mittlere Teilchengröße: 0,5 $\mu$m), 30 Gew.-% Bariumglaspulver (mittlere Teilchengröße: 0,5 $\mu$m).

Die DE 601 11 868 T2 beansprucht Dentalkomposite, um Kronen, Überzüge, direkte Füllungen, Inlays, Onlays und Schienen zu erzeugen. Die Dentalzusammensetzungen enthalten ein polymerisierbares Harz und 11 bis 80 Vol.- % Füllstoff, der im Wesentlichen aus einem gemahlenen Strukturfüllstoff und einem Nanofüllstoff besteht, wobei der gemahlene Strukturfüllstoff zwischen 10 Vol.-% und 70 Vol.-% des Komposit ausmacht und aus gemahlenen Teilchen mit einer durchschnittlichen Teilchengröße zwischen 0,05 und 0,5 $\mu$m besteht und wobei der gemahlene Strukturfüllstoff weniger als 50 Vol.-% an Teilchen über 0,5 $\mu$m im Durchmesser enthält und wobei der Nanofüllstoff zwischen 1,0 und 15 Vol.-% der Komposits ausmacht und im Wesentlichen aus diskreten, nicht aggregierten Teilchen mit einer durchschnittlichen Teilchengröße von weniger als 100 nm besteht. In den Beispielen liegt der Gesamtfüllstoffgehalt in Gew.- % zwischen 75 und 82,4 Gew.-%. Harz 1 umfasst Bis-GMA/TEGDMA und ethoxyliertes BPA-dimethacrylat mit 3,5 Ethoxygruppen pro Molekül im Gewichtsverhältnis 3,0/25/72. Harz 2 enthält ethoxyliertes BPA-dimethacrylat mit 2,0 Ethoxygruppen pro Molekül sowie HEDMA im Gewichtsverhältnis 90/10.

In der US 5,962,550 wird ein kunststoffmodifizierter Glasionomerzement als direktes Füllungsmaterial, als Stumpfaufbaumaterial und als ein Versiegelungsmaterial von Grübchen und Fissuren offenbart. In der Kunstharzphase weist dieses System in den experimentellen untersuchten Bindemittelzusammensetzungen u.a. folgende Gewichtsteile auf: Bis-MEPP/UDMA/HEMA (Hydroxyethylmethacrylat) 45/45/10, Bis-MEPP/UDMA/BG (Butandioldimethacrylat) 45/45/10, Bis-MEPP/UDMA/TEGDMA 33/38/29, Bis-MEPP/UDMA/HEMA 20/46/34.

Die US 4,616,073 beschreibt hoch fluorierte Methacrylatprepolymere, die in nichthydroxylierten Bis-GMA Systemen verwendet werden und sich zum Einsatz als Versiegelungsmaterial und Zement eignen sollen. Diese hydrophoben Zusammensetzungen sollen weitestgehend unempfindlich gegenüber einer chemischen Weichwerdung oder einem chemischen Abbau sein. Darüberhinaus sollen sie geringen Polymerisationsschrumpf und geringe Wasseraufnahme zeigen. In den Beispielen gibt es füllstofffreie Zusammensetzungen, in denen das Bis-EMA (2,2-Bis[(methacryloxyethyloxy)phenyl]propan) mit 2 Ethoxygruppen zusammen mit DMDMA (1,10-Decamethylendimethacrylat), PDFOMA (Pentadecafluorooctylmethacrylat) und BDMA (p-tert.-Butyl-N,N-dimethylanilin) in Gewichtsverhältnissen 44,31/44,31/11,15,0,23 sowie 44,25/44,25/11,15/0,35 sowie 49,25/41,25/9,30/0,20 sowie 45,91/45,91/7,98/0,20 verwendet wird. In den füllstoffhaltigen Zusammensetzungen wird das Bis-EMA in Mengen von 10 und 5,8 Gew.-% eingesetzt (Form Nr. 5 und 10B).

Die US 6,030,606 zielt unter anderem auch auf gehärtete Produkte wie Kronen, Brücken, Inlays, Onlays und Implantate. Die erfindungsgemäßen Zusammensetzungen, die ein ausgezeichnetes Eigenschaftsprofil wie eine hohe Materialfestigkeit und Härte aufweisen sollen, enthalten 10 - 30% einer Harzkomponente, die umfasst:
15 bis 45% BisEMA6, 15 bis 45% UDMA, 10 bis 40% Bis-GMA und 0 bis 10% TEGDMA. Die Anzahl der Ethoxygruppen beim BisEMA6 soll zwischen 5 und 8, bevorzugt bei 6 liegen.

Die DE 10 2015 220 373 A1 offenbart sowohl härtbare als auch gehärtete Dentalmaterialien und beansprucht ebenfalls Fräsrohlinge. Die hier beschriebenen Zusammensetzungen umfassen eine bimodale Glaszusammensetzung.

Die US 2014/0200284 A1 beschreibt radikalisch härtbare dentale Zusammensetzungen, deren Verwendung auch als Fräsrohlinge vorgesehen ist und deren E-Modul zwischen 10 und 14 GPa sein kann.

Im Stand der Technik zu den Fräsrohlingen wird zuvorderst auf die mechanischen Eigenschaften wie Biegefestigkeit und Härte sowie auf die ästhetischen Aspekte der Rohlinge hingearbeitet.

Trotz großer Fortschritte in der Materialentwicklung werden zunehmend höhere Ansprüche an moderne dentale Restaurationswerkstoffe gestellt. Dies gilt sowohl für die direkten Füllungsmaterialien als auch für indirekte Materialien wie z.B. CAD/CAM-gefertigte Restaurationen. Neben den steigenden ästhetischen Ansprüchen beispielsweise hinsichtlich der Farbe, der Transluzenz/Opazität und der Opaleszenz der Fräsrohlinge, gibt es auch weitergehende Anforderungen an die physikalischen Eigenschaften. So sollen die Materialien unter anderem eine hohe Festigkeit, eine niedrige Abrasion, eine gute Röntgensichtbarkeit und eine niedrige Wasseraufnahme aufweisen.

Besonders die Wasseraufnahme kann zu vielfältigen Problemen führen. So kommt es bei einer hohen Wasseraufnahme in der Regel vermehrt zu Verfärbungen, da mit dem Wasser oft auch Farbstoffe aufgenommen werden. Neben diesem ästhetischen Aspekt können durch eine erhöhte Wasseraufnahme aber auch die Esterbindungen der radikalisch gehärteten Harzmatrix hydrolytisch gespalten werden und so mechanische Parameter wie Festigkeit und Abrasionsbeständigkeit reduziert werden.

Die im Material auftretende Spannung wird im Allgemeinen durch den Spannungstensor ($\sigma_{ij}$) und die resultierende Verformung durch den Verzerrungstensor ($\varepsilon_{kl}$) beschrieben. Beides sind Tensoren 2. Stufe.

$$\sigma_{ij} = \begin{bmatrix} \sigma_{11} & \sigma_{12} & \sigma_{13} \\ \sigma_{21} & \sigma_{22} & \sigma_{23} \\ \sigma_{31} & \sigma_{32} & \sigma_{33} \end{bmatrix} \qquad \varepsilon_{kl} = \begin{bmatrix} \varepsilon_{11} & \varepsilon_{12} & \varepsilon_{13} \\ \varepsilon_{21} & \varepsilon_{22} & \varepsilon_{23} \\ \varepsilon_{31} & \varepsilon_{32} & \varepsilon_{33} \end{bmatrix} \quad (1)$$

Der Spannungstensor ist mit dem Verzerrungstensor über den Elastizitätstensor ($E_{ijkl}$) verknüpft, wobei der Elastizitätstensor ein Tensor 4. Stufe mit 81 Komponenten (i,j,k,l = 1,...,3) ist.

$$\sigma_{ij} = E_{ijkl}\varepsilon_{kl} \qquad (2)$$

Aufgrund der Symmetrie von Spannungs- und Verzerrungstensor reduziert sich die Zahl der unabhängigen Komponenten von $E_{ijkl}$ nach Überführung in $E_{IJ}$ jedoch auf 36. Damit lässt sich die elastische Konstante in einer 6x6-Matrix sowie die Spannung und die Verzerrung jeweils als sechskomponentige Vektoren darstellen.

$$\begin{bmatrix} \sigma_1 \\ \sigma_2 \\ \sigma_3 \\ \sigma_4 \\ \sigma_5 \\ \sigma_6 \end{bmatrix} = \begin{bmatrix} E_{11} & E_{12} & E_{13} & E_{14} & E_{15} & E_{16} \\ E_{21} & E_{22} & E_{23} & E_{24} & E_{25} & E_{26} \\ E_{31} & E_{32} & E_{33} & E_{34} & E_{35} & E_{36} \\ E_{41} & E_{42} & E_{43} & E_{44} & E_{45} & E_{46} \\ E_{51} & E_{52} & E_{53} & E_{54} & E_{55} & E_{56} \\ E_{61} & E_{62} & E_{63} & E_{64} & E_{65} & E_{66} \end{bmatrix} \begin{bmatrix} \varepsilon_1 \\ \varepsilon_2 \\ \varepsilon_3 \\ \varepsilon_4 \\ \varepsilon_5 \\ \varepsilon_6 \end{bmatrix} \qquad (3)$$

Vereinfacht ergibt sich somit der Zusammenhang zwischen der durch Wasseraufnahme auftretenden Spannung $(\sigma)$ und der daraus resultierenden Verformung $\varepsilon$ (Quellung) über den Elastizitätsmodul (E) als Proportionalitätskonstante:

$$\sigma = E\,\varepsilon \qquad (4)$$

bzw.

$$\varepsilon = \frac{\sigma}{E} \qquad (5)$$

Daraus ergibt sich, dass ein Material mit einem hohen Elastizitätsmodul in der Lage ist, auftretenden Spannungen besser entgegenzuwirken, so dass bei gleicher Spannung eine geringere Verformung resultiert.

Es wurde jetzt gefunden, dass für gehärtete Dentalkomposite die auftretende Verformung (Quellung) proportional zur Wasseraufnahme $(W_{SP})$ und außerdem umgekehrt proportional zum Elastizitätsmodul ist. Dies bedeutet, dass die auftretende Spannung proportional zur Wasseraufnahme ist.

$$\varepsilon \sim \sigma \sim W_{SP} \qquad (6)$$

und

$$\varepsilon \sim \frac{1}{E} \qquad (7)$$

$$\varepsilon \sim \frac{W_{SP}}{E} \qquad (8)$$

Abbildung 1 zeigt das Diagramm des Quotienten aus Wasseraufnahme/Elastizitätsmodul gegen die lineare Quellung für experimentelle Fräsrohlinge.

Abbildung 2 zeigt das Diagramm des Quotienten aus Wasseraufnahme/Elastizitätsmodul gegen die lineare Quellung für kommerzielle Fräsrohlinge.

Abbildung 3 zeigt die Darstellung einer idealisierten Krone zur Bestimmung der linearen Quellung.

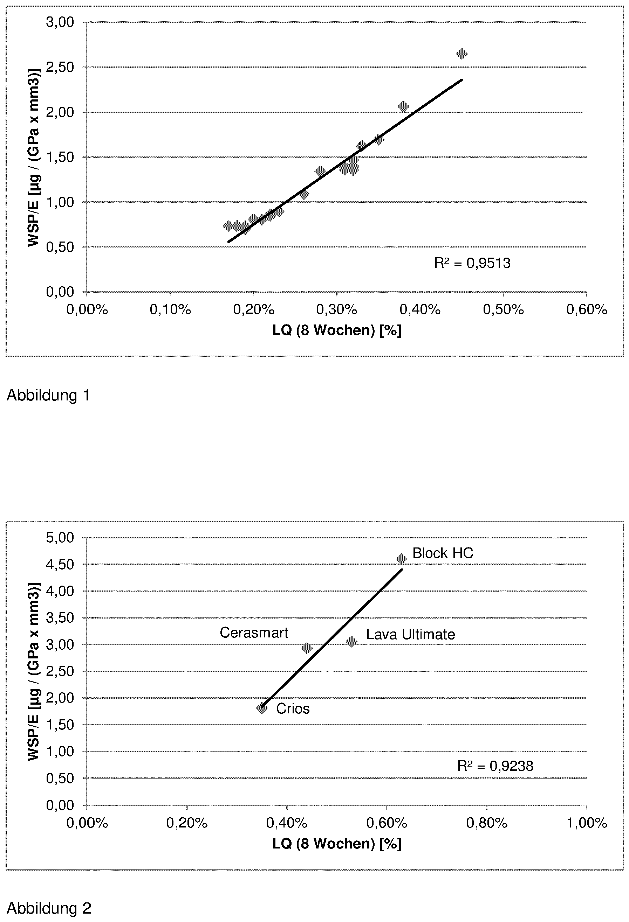

Aus den Abbildungen 1 und 2 ergibt sich eine gute Korrelation zwischen diesem Quotienten und der linearen Quellung. Die Daten aus diesen Abbildungen stammen aus den Beispielen (siehe experimenteller Teil).

Überraschend ist insbesondere, dass die Proportionalität nicht bloß zwischen der Quellung und der Wasseraufnahme besteht, sondern zwischen der Quellung und dem Quotienten aus Wasseraufnahme und Elastizitätsmodul. Materialien mit unterschiedlichen Elastizitätsmoduln zeigen also bei gleicher Wasseraufnahme unterschiedlich starke Quellungen.

Doch selbst der obige Befund einer strengen Proportionalität zwischen Wasseraufnahme und Quellung (bei konstantem E-Modul) ist insofern überraschend, da sich das hier erstmals aufgefundene Phänomen nicht allein durch das Volumen der aufgenommenen Wassermoleküle im Polymerisat erklären lässt, da analoge Versuche mit organischen Lösungsmitteln statt Wasser keine Proportionalität zwischen deren Aufnahme und einer Quellung des Materials zeigten. Vermutlich wirken die Wassermoleküle zerstörend auf vorhandene Überstrukturen wie der Sekundär- oder Tertiärstruktur im Polymerisat. Unter den extremen Bedingungen im Mundmilieu, insbesondere unter dem fortwährenden Einfluss von Speichel, kommt es zu einer Wasseraufnahme der fertigen Restauration. Wassermoleküle lagern sich im Polymernetzwerk ein, bilden dort Wasserstoffbrückenbindungen aus, beanspruchen Raum für sich und stören ausgebildete Überstrukturen. Dies führt zu Spannungen im Material. Es resultiert mikroskopisch eine Aufweitung des Polymernetzwerkes und makroskopisch eine Quellung der Restauration.

Beispielsweise führt die Quellung einer Krone zur Vergrößerung des Außen- und Innendurchmessers, da sich die gesamte Restauration durch die Quellung vergrößert. Dabei beträgt die relative lineare Quellung (in Prozent) etwa ein Drittel der relativen Volumenquellung (in Prozent). Durch die Vergrößerung des Innendurchmessers entsteht eine Zugkraft, die auf den Haftverbund der befestigten Krone ausgeübt wird. Übersteigen die Zugkräfte lokal die Haftung, kommt es an diesen Stellen zu Abrissen des Befestigungsmaterials und zu Randspaltbildung. Diese Stellen bergen ein erhöhtes Risiko für Ansiedlung von Bakterien und die Bildung von Sekundärkaries. Ist die Quellung groß genug oder die fortwährende Belastung der gebildeten Fehlstellen hoch genug, kommt es zum totalen Retentionsverlust der Restauration.

Aus Komposit-Fräsrohlingen hergestellte Restaurationen werden adhäsiv befestigt. Dabei werden die durch die Quellung auftretenden Spannungen über das Befestigungskomposit an den Haftverbund zur Zahnsubstanz weitergegeben. Typische adhäsive Befestigungskomposite weisen Elastizitätsmoduln von etwa 5 GPa auf (Journal of Prosthodontic Research 2010, 54, 59-64). Damit wirken bei einer Quellung von mehr als 0,25% Zugspannungen von mehr als 12,5 MPa auf den Haftverbund. Bei einer Quellung von mehr als 0,3% wirken Zugspannungen von mehr als 15 MPa. Bei höheren Quellungen und damit verbunden höheren Zugspannungen werden die Haftwerte des Befestigungsmaterials überschritten und es kommt zum Retentionsverlust.

Um retentionssichere und randspaltfreie prothetische Versorgungen zur Verfügung stellen zu können, war es somit Aufgabe der Erfindung, (möglichst) quellfreie Fräsrohlinge zu erhalten.

In umfangreichen eigenen Untersuchungen konnten wir zeigen, dass diese Werkstoffe dann zugänglich sind, wenn ihre Wasseraufnahme WSP kleiner/gleich 15 $\mu$g/mm$^3$, gemessen nach ISO 4049 und ihr E-Modul E größer/gleich 15 GPa, gemessen nach der ADA Spezifikation No. 27 und der Quotient Q aus WSP/E kleiner 1 $\mu$g/(GPa x mm$^3$) ist.

Dentale Fräsrohlinge mit den oben angegebenen Werten sind bislang im Stand der Technik nicht bekannt (siehe Tabelle 1 und Abbildung 2).

Völlig überraschend haben wir zudem gefunden, dass die obigen Voraussetzungen zum Erhalt (möglichst) quellfreier oder nur minimal quellender Polymerisate dann erreicht werden können, wenn der Gewichtsanteil von ethoxyliertem Bisphenol-A-Dimethacrylat mit einem durchschnittlichen Ethoxylierungsgrad von 2 bis 4 Ethoxygruppen pro Molekül größer als 40 Gewichtsprozent und kleiner als 50 Gewichtsprozent der eingesetzten Menge an radikalisch polymerisierbaren Monomeren ist und die Gesamtmasse an anorganischen Füllstoffen mindestens 83 Gewichtsprozent an der Gesamtzusammensetzung ausmacht. Dieser Befund ist insofern nicht zu erwarten gewesen, da mit einem ausgewiesen hydrophoben Monomeren wie HDDMA (Hexandioldimethacrylat) die obigen Werte der Wasseraufnahme nicht erreicht werden konnten. Der Fachmann hätte erwartet, dass durch die höhere Polarität der Ethoxybindung sowie des aromatischen Charakters der BPA-Ringstruktur eigentlich eine höhere Wasseraufnahme gegenüber einem reinen aliphatischen Kohlenwasserstoffgerüst wie dem von HDDMA erfolgen müsste, was aber nicht der Fall ist.

Die vorliegende Erfindung betrifft somit einen Hochleistungskompositblock, beziehungsweise einen dentalen Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der aus dem Polymerisat einer radikalisch härtbaren dentalen Zusammensetzung besteht, die umfasst

a) anorganische Füllstoffe, wobei die Gesamtmasse der anorganischen Füllstoffe mindestens 83 Gew.-%, bezogen auf die Gesamtmasse der Zusammensetzung, beträgt,

b) radikalisch polymerisierbare Monomere,

c) einen oder mehrere Initiatoren zur radikalischen Härtung sowie

d) optional Additive.

Ein bevorzugter dentaler Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der aus dem Polymerisat einer radikalisch härtbaren dentalen Zusammensetzung besteht, die umfasst

a) anorganische Füllstoffe, wobei die Gesamtmasse der anorganischen Füllstoffe mindestens 83 Gew.-%, bezogen auf die Gesamtmasse der Zusammensetzung, beträgt,

b) radikalisch polymerisierbare Monomere, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist,

c) einen oder mehrere Initiatoren zur radikalischen Härtung sowie

d) optional Additive.

Ein weiter bevorzugter Fräsrohling zur Herstellung permanenter indirekter Restaurationen im CAD/CAM Verfahren ist dadurch gekennzeichnet, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der aus dem Polymerisat einer radikalisch härtbaren dentalen Zusammensetzung besteht, die umfasst

a) anorganische Füllstoffe, wobei die Gesamtmasse der anorganischen Füllstoffe mindestens 83 Gew.-%, bezogen auf die Gesamtmasse der Zusammensetzung, beträgt und wobei die anorganischen Füllstoffe a) umfassen,

a1) eine Glaszusammensetzung und

a2) nicht aggregierte und nicht agglomerierte Kieselsäure mit einer mittleren Teilchengröße von nicht mehr als 80 nm sowie

b) radikalisch polymerisierbare Monomere, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist und

c) einen oder mehrere Initiatoren zur radikalischen Härtung sowie

d) optional Additive.

Ein ganz bevorzugter Fräsrohling zur Herstellung permanenter indirekter Restaurationen im CAD/CAM Verfahren ist dadurch gekennzeichnet, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der aus dem Polymerisat einer radikalisch härtbaren dentalen Zusammensetzung besteht, die umfasst

a) anorganische Füllstoffe, wobei die Gesamtmasse der anorganischen Füllstoffe mindestens 83 Gew.-%, bezogen auf die Gesamtmasse der Zusammensetzung, beträgt und wobei die anorganischen Füllstoffe a) umfassen,

a1) eine Glaszusammensetzung und

a2) nicht aggregierte und nicht agglomerierte Kieselsäure mit einer mittleren Teilchengröße von nicht mehr als 80 nm,

wobei die Glaszusammensetzung a1) eine erste Glaszusammensetzung a1a) mit einem D50-Wert von 0,4 - 1,0 $\mu$m, vorzugsweise von 0,5 - 0,9 $\mu$m, und eine zweite Glaszusammensetzung a1b) mit einem D50-Wert von 1,2 - 5,0 $\mu$m, vorzugsweise von 1,5 - 4,0 $\mu$m, umfasst und wobei das Massenverhältnis von a1a) zu a1b) zwischen 1 : 1,5 und 1 : 8, vorzugsweise zwischen 1 : 2 bis 1 : 5 und das Massenverhältnis von a2) zur Summe von a1a) und a1b) zwischen 1 : 3 und 1 : 6 liegt und das Verhältnis der mittleren Korngröße der ersten Mikropartikelfraktion a1a) zur mittleren Korngröße der zweiten Mikropartikelfraktion a1b) im Bereich von 1 : 1,5 bis 1 : 10, vorzugsweise 1 : 2 bis 1 : 5 liegt,

wobei der D75-Wert von a1a) kleiner ist als der D25-Wert von a1b) sowie

b) radikalisch polymerisierbare Monomere, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist und

c) einen oder mehrere Initiatoren zur radikalischen Härtung sowie

d) optional Additive.

Ein besonders bevorzugter Fräsrohling zur Herstellung permanenter indirekter Restaurationen im CAD/CAM Verfahren ist dadurch gekennzeichnet, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der aus dem Polymerisat einer radikalisch härtbaren dentalen Zusammensetzung besteht, die umfasst

a) anorganische Füllstoffe, wobei die Gesamtmasse der anorganischen Füllstoffe mindestens 83 Gew.-%, bezogen auf die Gesamtmasse der Zusammensetzung, beträgt und wobei die anorganischen Füllstoffe a) umfassen,

a1) eine Glaszusammensetzung und

a2) nicht aggregierte und nicht agglomerierte Kieselsäure mit einer mittleren Teilchengröße von nicht mehr als 80 nm,

wobei die Glaszusammensetzung a1) eine erste Glaszusammensetzung a1a) mit einem D50-Wert von 0,4 - 1,0 $\mu$m, vorzugsweise von 0,5 - 0,9 $\mu$m, und eine zweite Glaszusammensetzung a1b) mit einem D50-Wert von 1,2 - 5,0 $\mu$m, vorzugsweise von 1,5 - 4,0 $\mu$m, umfasst und wobei das Massenverhältnis von a1a) zu a1b) zwischen 1 : 1,5 und 1 : 8, vorzugsweise zwischen 1 : 2 bis 1 : 5 und das Massenverhältnis von a2) zur Summe von a1a) und a1b) zwischen 1 : 3 und 1 : 6 liegt und das Verhältnis der mittleren Korngröße der ersten Mikropartikelfraktion a1a) zur mittleren Korngröße der zweiten Mikropartikelfraktion a1b) im Bereich von 1 : 1,5 bis 1 : 10, vorzugsweise 1 : 2 bis 1 : 5 liegt,

wobei der D75-Wert von a1a) kleiner ist als der D25-Wert von a1b) und wobei der Anteil der nicht aggregierten und nicht agglomerierten Kieselsäure mit einer mittleren Teilchengröße von nicht mehr als 80 nm a2) größer als 11,86 Gew.-% und kleiner als 23 Gew.-%, bezogen auf die Gesamtzusammensetzung, ist sowie

b) radikalisch polymerisierbare Monomere, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist, wobei die Menge an radikalisch polymerisierbaren Monomeren b) in einem Mengenbereich von höchstens 16,7 Gew.-%, bezogen auf die Gesamtzusammensetzung, liegt und

c) einen oder mehrere Initiatoren zur radikalischen Härtung von 0,2 bis 5 Gew.-%, bezogen auf die Gesamtzusammensetzung, sowie

d) Additive in einem Mengenbereich von 0,001 Gew.-% bis 2 Gew.-%, bezogen auf die Gesamtzusammensetzung.

Anders ausgedrückt betrifft die vorliegende Erfindung somit einen dentalen Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa $\times$ mm$^3$) aufweist und der enthält

a) anorganische Füllstoffe, wobei die Gesamtmasse der anorganischen Füllstoffe mindestens 83 Gew.-%, bezogen auf die Gesamtmasse des Fräsrohlings, beträgt sowie

b) polymerisierte Anteile an radikalisch polymerisierbaren Monomeren.

Anders ausgedrückt betrifft die vorliegende Erfindung somit einen bevorzugten dentalen Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa $\times$ mm$^3$) aufweist und der enthält

a) anorganische Füllstoffe, wobei die Gesamtmasse der anorganischen Füllstoffe mindestens 83 Gew.-%, bezogen auf die Gesamtmasse des Fräsrohlings, beträgt sowie

b) polymerisierte Anteile an radikalisch polymerisierbaren Monomeren, die difunktionelle (Meth)acrylate umfassen, wobei der Gewichtsanteil an polymerisiertem ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist.

Anders ausgedrückt betrifft die vorliegende Erfindung somit einen weiteren bevorzugten Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und enthält

a) anorganische Füllstoffe, wobei die Gesamtmasse der anorganischen Füllstoffe mindestens 83 Gew.-%, bezogen auf die Gesamtmasse des Fräsohlings, beträgt und wobei die anorganischen Füllstoffe a) umfassen,

a1) eine Glaszusammensetzung und

a2) nicht aggregierte und nicht agglomerierte Kieselsäure mit einer mittleren Teilchengröße von nicht mehr als 80 nm sowie

b) polymerisierte Anteile an radikalisch polymerisierbaren Monomeren, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist.

Anders ausgedrückt betrifft die vorliegende Erfindung somit einen ganz bevorzugten Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der enthält

a) anorganische Füllstoffe, wobei die Gesamtmasse der anorganischen Füllstoffe mindestens 83 Gew.-%, bezogen auf die Gesamtmasse des Fräsrohlings, beträgt und wobei die anorganischen Füllstoffe a) umfassen,

a1) eine Glaszusammensetzung und

a2) nicht aggregierte und nicht agglomerierte Kieselsäure mit einer mittleren Teilchengröße von nicht mehr als 80 nm,

wobei die Glaszusammensetzung a1) eine erste Glaszusammensetzung a1a) mit einem D50-Wert von 0,4 - 1,0 $\mu$m, vorzugsweise von 0,5 - 0,9 $\mu$m, und eine zweite Glaszusammensetzung a1b) mit einem D50-Wert von 1,2 - 5,0 $\mu$m, vorzugsweise von 1,5 - 4,0 $\mu$m, umfasst und wobei das Massenverhältnis von a1a) zu a1b) zwischen 1 : 1,5 und 1 : 8, vorzugsweise zwischen 1 : 2 bis 1 : 5 und das Massenverhältnis von a2) zur Summe von a1a) und a1b) zwischen 1 : 3 und 1 : 6 liegt und das Verhältnis der mittleren Korngröße der ersten Mikropartikelfraktion a1a) zur mittleren Korngröße der zweiten Mikropartikelfraktion a1b) im Bereich von 1 : 1,5 bis 1 : 10, vorzugsweise 1 : 2 bis 1 : 5 liegt,

wobei der D75-Wert von a1a) kleiner ist als der D25-Wert von a1b) sowie

b) polymerisierte Anteile an radikalisch polymerisierbaren Monomeren, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist.

Anders ausgedrückt betrifft die vorliegende Erfindung somit einen besonders bevorzugten Fräsrohling zur Herstellung permanenter indirekter Restaurationen im CAD/CAM Verfahren, der dadurch gekennzeichnet ist, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und der enthält

a) anorganische Füllstoffe, wobei die Gesamtmasse der anorganischen Füllstoffe mindestens 83 Gew.-%, bezogen auf die Gesamtmasse des Fräsrohlings, beträgt und wobei die anorganischen Füllstoffe a) umfassen,

a1) eine Glaszusammensetzung und

a2) nicht aggregierte und nicht agglomerierte Kieselsäure mit einer mittleren Teilchengröße von nicht mehr als 80 nm,

wobei die Glaszusammensetzung a1) eine erste Glaszusammensetzung a1a) mit einem D50-Wert von 0,4 - 1,0 μm, vorzugsweise von 0,5 - 0,9 μm, und eine zweite Glaszusammensetzung a1b) mit einem D50-Wert von 1,2 - 5,0 μm, vorzugsweise von 1,5 - 4,0 μm, umfasst und wobei das Massenverhältnis von a1a) zu a1b) zwischen 1 : 1,5 und 1 : 8, vorzugsweise zwischen 1 : 2 bis 1 : 5 und das Massenverhältnis von a2) zur Summe von a1a) und a1b) zwischen 1 : 3 und 1 : 6 liegt und das Verhältnis der mittleren Korngröße der ersten Mikropartikelfraktion a1a) zur mittleren Korngröße der zweiten Mikropartikelfraktion a1b) im Bereich von 1 : 1,5 bis 1 : 10, vorzugsweise 1 : 2 bis 1 : 5 liegt,

wobei der D75-Wert von a1a) kleiner ist als der D25-Wert von a1b) und wobei der Anteil der nicht aggregierten und nicht agglomerierten Kieselsäure mit einer mittleren Teilchengröße von nicht mehr als 80 nm a2) größer als 11,86 Gew.-% und kleiner als 23 Gew.-%, bezogen auf die Gesamtzusammensetzung, ist,

b) polymerisierte Anteile an radikalisch polymerisierbaren Monomeren, die difunktionelle (Meth)acrylate umfassen, wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist, wobei die Menge an radikalisch polymerisierbaren Monomeren b) in einem Mengenbereich von höchstens 16,7 Gew.-%, bezogen auf die Gesamtzusammensetzung, liegt.

a) anorganische Füllstoffe

Der erfindungsgemäße Fräsohling umfasst anorganische Füllstoffe in einer Menge von mindestens 83 Gew.-%, bezogen auf die Gesamtzusammensetzung. Die anorganischen Füllstoffe werden bevorzugt als Mischungen eingesetzt. Zur Optimierung der Produkteigenschaften werden die anorganischen Füllstoffe in unterschiedlichen Korngrößen in die Rezepturen eingebracht, wobei sie vorzugsweise eine multimodale, ganz bevorzugt eine bimodale Verteilung aufweisen.

Als anorganische Füllstoffe können kompakte Gläser und unterschiedliche Kieselsäuren in verschiedenen Größen und Zuständen (monodispers, polydispers) zum Einsatz kommen.

Geeignete anorganische Bestandteile sind beispielsweise amorphe Materialien auf der Basis von Mischoxiden aus $SiO_2$, $ZrO_2$ und/oder $TiO_2$ sowie Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluraluminiumsilikatgläser, Oxide von Aluminium oder Silicium, Zeolithe, Apatit, Zirkonsilikate, schwerlösliche Metallsalze wie Bariumsulfat oder Calciumfluorid sowie röntgenopake Füllstoffe wie Ytterbiumfluorid.

In einer bevorzugten Ausführung enthält ein erfindungsgemäßer Fräsrohling Barium-Aluminium-Borsilikatgläser.

Zum besseren Einbau in die Polymermatrix können die Füllstoffe organisch oberflächenmodifiziert sein. Beispielhaft genannt sei die Oberflächenbehandlung der Füllstoffe mit einem Silan. Als Haftvermittler eignet sich besonders das Methacryloxypropyltri methoxysilan.

In einer bevorzugten Ausführungsform enthält ein erfindungsgemäßer Fräsrohling oberflächenbehandelte Barium-Aluminium-Borsilikatgläser, vorzugsweise silanisierte Barium-Aluminium-Borsilikatgläser und am meisten bevorzugt mit Methacryloxypropyltrimethoxysilan behandelte Barium-Aluminium-Borsilikatgläser.

Die erfindungsgemäßen Fräsrohlinge können unterschiedliche Kieselsäuren enthalten.

Bevorzugt enthalten die erfindungsgemäßen Fräsrohlinge nanoskalige Kieselsäuren. Bei den nanoskaligen Kieselsäuren handelt es sich um Partikel mit einer mittleren Teilchengröße von nicht mehr als 80 nm. Die Herstellung der nanoskaligen Kieselsäuren erfolgt auf bekannte Weise, z.B. durch Flammpyrolyse, Plasmaverfahren, Gasphasenkondensation, Kolloidtechniken, Präzipitationsverfahren, Sol-Gel Verfahren, etc.

In einer bevorzugten Ausgestaltung liegen die nanoskaligen Kieselsäuren in nicht agglomerierter und nicht aggregierter Form vor, vorzugsweise in monodisperser Form.

Um eine gute Einbindung der Nanopartikel in die Polymermatrix einer radikalisch härtbaren dentalen Zusammensetzung zu ermöglichen, sind die Oberflächen der nanoskaligen Kieselsäuren ebenfalls organisch oberflächenmodifiziert, d.h. ihre Oberflächen weisen organische Strukturelemente auf. Beispielhaft genannt sei die Oberflächenbehandlung der Füllstoffe mit einem Silan. Als Haftvermittler eignet sich auch hier besonders das Methacryloxypropyltri methoxysilan.

In einer bevorzugten Ausführungsform enthält ein erfindungsgemäßer Fräsrohling oberflächenbehandelte nanoskalige, nicht agglomerierte und nicht aggregierte Kieselsäureteilchen mit einer mittleren Partikelgröße von nicht mehr als 80 nm, vorzugsweise silanisierte nanoskalige, nicht agglomerierte und nicht aggregierte Teilchen mit einer mittleren Partikelgröße von nicht mehr als 80 nm und am meisten bevorzugt mit Methacryloxypropyltrimethoxysilan behandelte, nanoskalige, nicht agglomerierte und nicht aggregierte Kieselsäureteilchen mit einer mittleren Partikelgröße von nicht mehr als 80 nm. Kommerziell erhältliche nanoskalige, nicht-agglomerierte und nicht aggregierte Kieselsole, die eingesetzt werden können, sind beispielsweise unter der Bezeichnung "NALCO COLLOIDAL SILICAS" (Nalco Chemical Co.), "Ludox colloidal silica" (Grace) oder "Highlink OG (Clariant) im Handel.

In einer bevorzugten Ausgestaltung umfasst der Füllstoffanteil des Fräsrohlings eine Mischung eines ersten Füllstoffs a2) in Form nicht agglomerierter, nicht aggregierter, organisch oberflächenmodifizierter Nanopartikel mit einer mittleren Partikelgröße kleiner 80 nm und eines zweiten Füllstoffs a1) in Form von Mikropartikeln mit einer mittleren Partikelgröße im Bereich von 0,4 $\mu$m bis 5 $\mu$m. Durch die Kombination von a2) Nanopartikeln und a1) Mikropartikeln in der Polymermatrix wird eine vollständige und gleichmäßige Volumenfüllung des Kompositmaterials erzielt.

Der Anteil organisch oberflächenmodifizierter Nanopartikel in einem erfindungsgemäßen Fräsrohling mit einer mittleren Partikelgröße kleiner 80 nm ist größer als 11,86 Gew.-% und kleiner als 23 Gew.-%, bezogen auf die Gesamtzusammensetzung. In eigenen Untersuchungen hat sich gezeigt, dass bei einem Gehalt von 11,86 Gew.-% oder weniger oder bei einem Gehalt von 23 Gew.-% und mehr an nicht agglomerierten und nicht aggregierten, organisch oberflächenmodifizierten Nanopartikeln mit einer mittleren Partikelgröße kleiner 80 nm der Fräsrohling nicht mehr ausreichend quellfest ist.

Innerhalb des Fräsrohlings bewirken die Mikropartikel eine weitgehende gleichmäßige Füllung des Volumens, wobei die verbleibenden Hohlräume zwischen den Mikropartikeln durch die oben beschriebenen Nanopartikel (Komponente a2)) zumindest teilweise gefüllt werden. Unter Mikropartikeln werden in Zusammenhang mit der vorliegenden Erfindung Partikel mit einer mittleren Partikelgröße von 400 nm bis 5 $\mu$m verstanden.

Die Mikropartikel der Komponente a1) weisen vorzugsweise eine bimodale Partikelgrößenverteilung auf. Mikropartikel mit einer bimodalen Partikelgrößenverteilung sind deshalb bevorzugt, da mit ihnen eine vollständigere Volumenfüllung erreichbar ist als bei allgemeiner Verwendung von Mikropartikeln mit monomodaler Partikelgrößenverteilung. Bei Vorliegen einer bimodalen Partikelgrößenverteilung bewirken die Partikel der Fraktionen mit der größeren Partikelgröße eine grobe Ausfüllung des Volumens, während die Partikel der Fraktion mit der kleineren Partikelgröße soweit möglich die Bereiche zwischen den Partikeln der Fraktionen mit der größeren Partikelgröße ausfüllen werden. Die noch verbleibenden Hohlräume werden wie oben beschrieben durch Nanopartikel gefüllt.

Der erfindungsgemäße Fräsrohling umfasst eine Komponente a1), welche eine erste Mikropartikelfraktionen a1a), die jeweils eine mittlere Partikelgröße im Bereich von 0,4 $\mu$m bis 1 $\mu$m, vorzugsweise von 0,5 $\mu$m bis 0,9 $\mu$m und eine zweite Mikropartikelfraktionen a1b), die jeweils eine mittlere Partikelgröße im Bereich von 1,2 $\mu$m bis 5,0 $\mu$m, vorzugsweise von 1,5 $\mu$m bis 4,0 $\mu$m, besitzen.

Das Verhältnis der Gesamtmasse der ersten Mikropartikelfraktion zur Gesamtmasse der zweiten Mikropartikelfraktion liegt im Bereich von 1 : 1,5 bis 1 : 8, vorzugsweise im Bereich von 1 : 2 bis 1 : 5.

b) radikalisch polymerisierbare Monomere/radikalisch polymerisierte Monomere

Der erfindungsgemäße dentale Fräsrohling umfasst Anteile radikalisch polymerisierter Monomere oder radikalisch polymerisierbarer Monomere des Polymerisats einer radikalisch härtbaren Zusammensetzung in einer Menge von höchstens 16,7 Gew.-%, bezogen auf die Gesamtzusammensetzung.

Die radikalisch polymerisierbaren Monomere des Polymerisats einer radikalisch härtbaren Zusammensetzung oder die Anteile radikalisch polymerisierter Monomere können, ohne darauf beschränkt zu sein, die in der Dentalchemie üblicherweise in Kompositmaterialien eingesetzten (Meth)acrylatmonomere sein.

In der Patentliteratur ist eine Vielzahl an Verbindungen genannt (beispielsweise auch in der DE 39 41 629 A1), die allesamt Diester der Acryl- oder Methacrylsäure sind und sich zum Einsatz in einem Polymerisat der vorliegenden Erfindung eignen.

In einer bevorzugten Ausführung enthält Bestandteil (b) ein oder mehrere Monomere gewählt aus der Gruppe bestehend aus Ethylenglykoldimethacrylat (EGDMA), 1,6-Hexandioldimethacrylat (HDDMA), Triethylenglykoldimethacrylat (TEGDMA), 1,10-Decandioldimethacrylat (DEDMA), 1,12-Dodecandioldimethacrylat (DODMA), ethoxyliertes Bisphenol-A-dimethacrylat, ethoxyliertes Bisphenol-A-dimethacrylat, wobei das Bisphenol mit 2 bis 4 mol Ethylenoxid umgesetzt wird und das Zwischenprodukt dann mit 2 mol Methacrylsäure abgesättigt wird, Polyethylenglykoldimethacrylat (PEGDMA), 7,7,9-Trimethyl-4,13-dioxo-3,14-dioxa-5,12-diazahexadecan-1,16-dioxydimethacrylat (UDMA), Butandioldimethacrylat, Tetraethylenglykoldimethacrylat, Neopentylglykoldimethacrylat sowie Bisphenol-A-Glycidyl-Methacrylat (Bis-GMA).

Verwendbar sind auch die entsprechenden Dimethacrylate bzw. Diacrylate des Dihydroxymethyltricyclo[5.2.1.0$^{2,6}$]decans, wie sie in den Druckschriften DE 1816823, DE 2419887, DE 2406557, DE 2931926, DE 3522005, DE 3522006, DE 3703120, DE 102005021332, DE 102005053775, DE 102006060983, DE 69935794 und DE 102007034457 beschrieben sind.

c) Initiatoren

Der erfindungsgemäße Fräsrohling ist entweder durch Strahlenhärtung (photochemisch) und/oder durch chemische Härtung (Redoxreaktion) und/oder thermisch herstellbar. Bevorzugt ist die thermische Härtung, die beispielsweise durch Peroxidzerfall herbeigeführt wird.

Beispiele für geeignete Photosensibilisatoren sind alpha-Diketone, Benzoinalkylether, Thioxanthone, Benzophenone, Acylphosphinoxide, Acylgermanium-Verbindungen, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1, oder in der DE 39 41 629 C2.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2.

Weitere geeignete Initiatoren sowie Initiatorkombinationen sind in der DE 601 16 142 beschrieben.

Geeignete Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer radikalisch härtbaren dentalen Zusammensetzung bewirken können.

Das Absorptionsmaximum von Campherchinon (CQ) liegt bei ca. 470 nm und somit im Bereich des blauen Lichts. Campherchinon (CQ) zählt zu den PI$_2$-Initiatoren und wird regelmäßig zusammen mit einem Coinitiator eingesetzt.

Ein geeignetes Katalysatorsystem enthält die Kombination eines alpha-Diketons und eines aromatischen tertiären Amins, bevorzugt ist die Kombination von Campherchinon (CQ) und Ethyl-p-N,N-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyldiphenylphosphinoxid. Bezüglich der Strukturen geeigneter Phosphinoxide wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen.

Die in diesen Druckschriften angegebenen Phosphinoxide eigenen sich besonders allein oder in Kombination mit dem System "alpha-Diketon/Amin" als Photopolymerisationsinitiatorsystem.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben.

Dem Fachmann sind diverse Initiatoren für eine chemische Aushärtung bekannt. Es sei insoweit exemplarisch auf die EP 1 720 506 verwiesen. Initiatoren zur chemischen Aushärtung werden auch in den oben bereits erwähnten Druckschriften DE 10 2006 019 092 sowie in der DE 39 41 629 beschrieben.

Bevorzugte Initiatoren zur chemischen Härtung sind Dibenzoylperoxid, Dilauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Dualhärtende Systeme umfassen eine Kombination aus Photoinitiatoren und Initiatoren zur chemischen Aushärtung.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in der EP 1 839 640 sowie in DE 1495520, WO 02/092021 oder in WO 02/092023.

Anstelle der Barbitursäuren können auch deren Salze verwendet werden. Beispiele hierzu finden sich in den folgenden Dokumenten: EP 1 872 767, EP 2 070 506, EP 1 881 010, DE 10 2007 050 763, US 6,288,138, DE 11 2006 001 049, US 7,214,726 sowie EP 2 070 935.

Geeignete Malonylsulfamide sind in der EP 0 059 451 beschrieben. Bevorzugte Verbindungen sind dabei 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Werden die Peroxide erwärmt, so zerfallen sie und bilden freie Radikale, die befähigt sind, die Polymerisation zu starten. Das am weitesten verbreitete System zur thermischen Polymerisation ist die Verwendung von Dibenzoylperoxid. Weitere thermische Initiatoren sind Ketonperoxide, Peroxyketale, Hydroperoxide, Dialkylperoxide, Diacylperoxide, Peroxyester und Peroxydicarbonate wie Dicumylperoxid, Chlorbenzoylperoxid, t-Butylperbenzoat Dilauroylperoxid, Cumolhydroperoxid, 3,5,5-Trimethylhexansäure-tert.-butylperoxyester sowie Azoverbindungen wie 2,2'-Azobisisobutyronitril, 2,2'-Azobis-2,4-dimethylvaleronitril, 2,2'-Azobis-1-cyclohexancabonitril oder Dimethyl-2-2'-azobisisobutyrat. Auch Substanzen wie Natrium- oder Kaliumpersulfat zerfallen thermisch und sind in diesem Zusammenhang geeignete Verbindungen. Diese Substanzen können einzeln oder in Mischungen untereinander verwendet werden. Hierzu müssen die radikalisch härtbaren dentalen Zusammensetzungen lediglich auf die vom Hersteller angegebene Zerfallstemperatur der jeweiligen Peroxide erwärmt werden. Vorteilhafterweise werden die radikalisch härtbaren Zusammensetzungen auf eine Temperatur oberhalb der Zerfallstemperatur erwärmt und dort für einige Zeit belassen, damit das Polymerisat Zeit zur Relaxation hat. Der Fachmann findet die optimale Temperatur dadurch, dass er die Temperatur zur Härtung sukzessive bis zu dem Punkt erhöht, an dem das Polymerisat keine wesentlichen Verbesserungen an den an ihm gemessenen wichtigen Parameter wie Biegefestigkeit, E-Modul und Wasseraufnahme aufweist.

Vorzugsweise wird die thermische Härtung so ausgeführt, dass die radikalisch härtbare Zusammensetzung in eine Blockform überführt wird, wo sie bei Temperaturen von 80°C bis 150°C und bei einem Druck von 100 bis 300 bar ausgehärtet wird.

d) Additive

Ein erfindungsgemäßer Fräsrohling umfasst in manchen Fällen ein oder mehrere weitere(s) Additiv(e).

Diese Additive können verschiedene Funktionen haben. Übliche Additive für den Einsatz in dentalen Werkstoffen sind dem Fachmann bekannt, je nach gewünschter Funktion wird er das oder die geeigneten Additive auswählen. Beispielhaft sind im Folgenden typische Additive und ihre Funktionen beschrieben.

UV-Absorber, die beispielsweise durch ihre konjugierten Doppelbindungssysteme und aromatischen Ringe befähigt sind, UV Strahlung zu absorbieren, sind in manchen Fällen Bestandteil eines erfindungsgemäßen Fräsrohlings. Beispiele an UV-Absorbern sind 2-Hydroxy-4-methoxybenzophenon, Salicylsäurephenylester 3-(2'-Hydroxy-5'-methylphenyl)benzotriazol oder Diethyl-2,5-dihydroxyterephthalat. Die Polymerisate enthalten diese Additive, um ihre Farbstabilität zu gewährleisten.

Da die Zähne möglichst naturgetreu wiederherzustellen sind, ist es notwendig, dentale Fräsrohlinge in den unterschiedlichsten Farbtönen bereitzustellen. Man benutzt zu diesem Zweck in der Regel anorganische Farbstoffe sowie organische Pigmente in sehr geringen Mengen.

Weitere optionale Additive sind dentale Arzneimittel, Mikrobizide, vorzugsweise Bakterizide oder Fluoreszenzmittel, die ebenfalls eingesetzt werden, um das natürliche Erscheinungsbild von Zähnen abzubilden.

Die vorliegende Erfindung betrifft außerdem ein Verfahren zur Herstellung eines dentalen Fräsrohlings zur Herstellung permanenter indirekter Restaurationen im CAD/CAM Verfahren dadurch gekennzeichnet, dass er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist mit den folgenden Schritten:

- Bereitstellen radikalisch polymerisierbarer Monomeren,

- Bereitstellen anorganischer Füllstoffe in einer Menge von wenigstens 83 Gew.-%, bezogen auf die Gesamtmasse der Zusammensetzung,

- Bereitstellen eines Polymerisationsinitiators,

- gegebenenfalls Bereitstellen von Additiven,

- homogenes Mischen der Komponenten und

- Polymerisieren der Mischung.

Nachdem aus dem Rohling beispielsweise eine Krone im CAD/CAM Verfahren gefräst wurde und der Zahnstumpf präpariert ist, wird der Zahnarzt vorzugsweise die innere Oberfläche der Krone mittels Sandstrahlen aufrauen, dann säubern und primen. Er wird dann das Bonding auf den Stumpf auftragen und härten und schließlich einen Befestigungszement in die Krone füllen und diese auf den Stumpf setzten.

Vorzugsweise werden die Fräsrohlinge somit als Bestandteil eines erfindungsgemäßen Kits eingesetzt. Die vorliegende Erfindung betrifft somit auch ein Kit, umfassend

- erfindungsgemäße Fräsrohlinge in unterschiedlichen Farben,

- wenigstens einen Primer,

- wenigstens ein dentales Adhäsiv,

- wenigstens einen Befestigungszement sowie

- optional weiteres Zubehör wie Pinsel, Poliermittel und Mischkanülen.

Beispiele:
Abkürzungen:

| | |
|---|---|
| Bis-EMA2,6: | Ethoxyliertes Bisphenol A-Dimethacrylat mit durchschnittlich 2,6 Ethylenoxideinheiten |
| Bis-EMA4: | Ethoxyliertes Bisphenol A-Dimethacrylat mit durchschnittlich 4 Ethylenoxideinheiten |
| Bis-EMA6: | Ethoxyliertes Bisphenol A-Dimethacrylat mit durchschnittlich 6 Ethylenoxideinheiten |
| Bis-EMA10: | Ethoxyliertes Bisphenol A-Dimethacrylat mit durchschnittlich 10 Ethylenoxideinheiten |
| TCDDMA: | Bis(methacryloyloxymethyl)tricyclo[5.2.1.0$^{2,6}$]decan |
| UDMA: | 7,7,9-Trimethyl-4, 13-dioxo-3, 14-dioxa-5, 12-diazahexadecan-1, 16-dioxydimethacrylat |
| TEGDMA: | Triethylenglycoldimethacrylat |
| HDDMA: | 1,6-Hexandioldimethacrylat |
| DODMA: | 1,12-Dodecandioldimethacrylat |
| Dentalglas 1: | Barium-Aluminium-Borosilikat-Glas (D50 0,8 $\mu$m / D25 0,5 $\mu$m / D75 1,0 $\mu$m) |
| Dentalglas 2: | Barium-Aluminium-Borosilikat-Glas (D50 2,7 $\mu$m / D25 1,4 $\mu$m / D75 6,1 $\mu$m) |
| Dentalglas 3: | Barium-Aluminium-Borosilikat-Glas (D50 1,1 $\mu$m / D25 0,7 $\mu$m / D75 1,4 $\mu$m) |
| Dentalglas 4: | Barium-Aluminium-Borosilikat-Glas (D50 1,4 $\mu$m / D25 1,1 $\mu$m / D75 2,1 $\mu$m) |
| Dentalglas 5: | Barium-Aluminium-Borosilikat-Glas (D50 1,4 $\mu$m / D25 0,8 $\mu$m / D75 2,9 $\mu$m) |
| Nano-SiO$_2$: | nicht agglomerierte, nicht aggregierte Kieselsäure (D50 40 nm) |
| BPO: | Dibenzoylperoxid |

Herstellung der Komposit-Pasten:
Die einzelnen Komponenten wurden entsprechend der in den Tabellen 2 bis 13 angegebenen Anteile eingewogen, für 30 Minuten bei 50 U/min an einem Laborkneter (PC Laborsystem, Magden CH) homogenisiert und anschließend am Laborkneter für 15 Minuten bei 50 U/min und -0,85 bar entlüftet.

Herstellung der Komposit-Blöcke:
Zur Herstellung der Komposit-Blöcke wurden die einzelnen Pasten in Formen (15 mm x 15 mm x 20 mm) gefüllt. Die Aushärtung erfolgte isostatisch bei 250 bar und folgendem Temperaturprogramm (20 °C - 2 °C/min - 120 °C (30 min) - 5 °C/min - 20 °C)

Biaxiale Biegefestigkeit (BBF): Die Biaxiale Biegefestigkeit wurde analog DIN EN ISO 6872:2009 (7.3.3) bestimmt. Dazu wurden aus den Komposit-Blöcken zunächst in einer 5-Achs Fräsmaschine (250i, imes-icore GmbH) Zylindern mit einem Durchmesser von 14 mm geschliffen. Aus diesen Zylindern wurden dann mit einer Hochgeschwindigkeitssäge (IsoMet 4000, Buehler) Scheiben mit einer Dicke von 1,2 mm hergestellt, entgratet, geschliffen und poliert. Die Proben wurden mit einer Traversengeschwindigkeit von 1 mm/min bis zum Bruch belastet und die biaxiale Biegefestigkeit entsprechend der unter 7.3.3.4 angegebenen Formel berechnet. Als Poisson'sche Querkontraktionszahl wurde ein Wert von 0,25 eingesetzt.

3-Punkt-Biegefestigkeit (3PBF): Die Biegefestigkeit wurde analog DIN EN ISO 6872:2009 (7.3.2) bei einer Stützweite von 12 mm und einem Auflagerollendurchmesser von 2 mm bestimmt. Dazu wurden aus den Komposit-Blöcken Probekörper mit einer Breite von 4 mm, einer Dicke von 1,2 mm und einer Länge von 18 mm mit einer Hochgeschwindigkeitssäge (IsoMet 4000, Buehler) hergestellt, entgratet, geschliffen und poliert. Die Proben wurden mit einer Traversengeschwindigkeit von 1 mm/min bis zum Bruch belastet und die 3-Punkt-Biegefestigkeit entsprechend der unter 7.3.2.4.1 angegebenen Formel berechnet.

Elastizitätsmodul (E): Der Elastizitätsmodul wurde analog der Berechnung in ADA Spec. No. 27:1993 (7.8.4.2) als Steigung der Spannung-Dehnungs-Kurve der 3-Punkt-Biegefestigkeitsmessung im linear-elastischen Bereich ermittelt.

$$E = \frac{3\,L}{4\,b\,h^3}\frac{\Delta F}{\Delta d}$$

L:     Stützweite

b:     Probenbreite

h:     Probendicke

$\Delta d$:     Deformation im linear-elastischen Bereich

$\Delta F$:     Kraftänderung bei einer Deformation $\Delta d$

Wasseraufnahme ($W_{SP}$): Die Wasseraufnahme wurde analog DIN EN ISO 4049:2010 (7.12) bestimmt. Dazu wurden aus den Komposit-Blöcken Probekörper mit einer Länge von 14,7 mm, einer Breite von 14,7 mm und einer Dicke von 0,5 mm mit einer Hochgeschwindigkeitssäge (IsoMet 4000, Buehler) hergestellt, entgratet, geschliffen und poliert. Die Probekörper wurden im Exsikkator bei 37 °C bis zur Massenkonstanz getrocknet und die Masse ($m_1$) auf 0,1 mg, sowie die Länge, die Breite und die Dicke auf 0,01 mm genau bestimmt. Anschließend wurden die Probekörper für 7 Tage bei 37 °C in Wasser gelagert. Nach 7 Tagen wurden die Probekörper herausgenommen, mit Wasser abgespült, abgetupft, für 15 Sekunden in der Luft hin- und hergeschwenkt und 1 Minute nach dem Herausnehmen aus dem Wasser auf 0,1 mg genau gewogen ($m_2$). Nach dieser Wägung werden die Probekörper im Exsikkator bei 37 °C bis zur Massenkonstanz getrocknet und die Masse ($m_3$) auf 0,1 mg bestimmt. Die Wasseraufnahme wurde entsprechend der unter 7.12.4.1 angegebenen Formel berechnet.

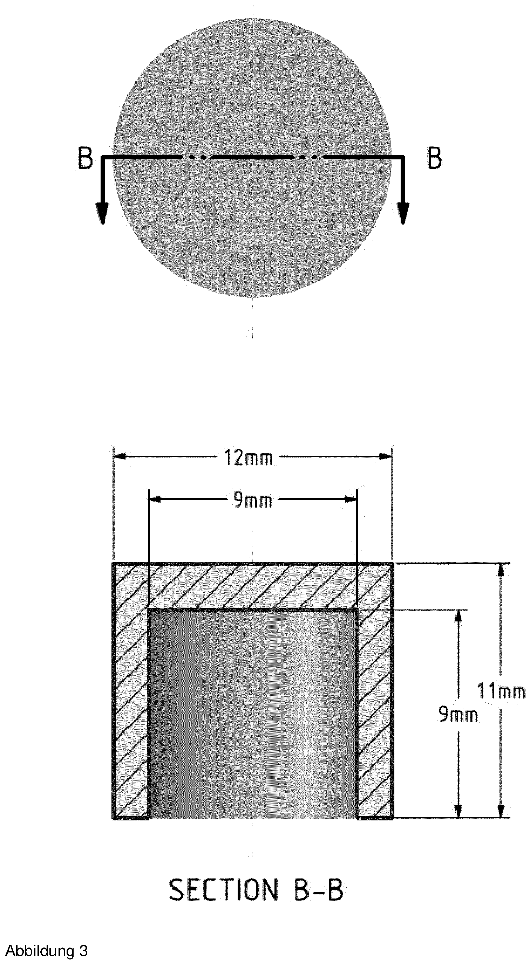

Lineare Quellung (LQ): In einer 5-Achs Fräsmaschine (250i, imes-icore GmbH) wurden idealisierte Kronen als Probekörper aus den Komposit-Blöcken hergestellt. Diese idealisierten Kronen sind einseitig geschlossene Hohlzylinder (vgl. Abbildung 3). Die Höhe beträgt 11 mm, der Außendurchmesser 12 mm und der Innendurchmesser 9 mm. Dies entspricht einer Wandstärke von 1,5 mm. Die Stärke der Deckplatte beträgt 2 mm. Anschließend wurden die Kronen entgratet, geschliffen und poliert. Die Probekörper wurden im Exsikkator bei 37 °C bis zur Massenkonstanz getrocknet und der Innendurchmesser an der Zylinderbasis an zwei Stellen orthogonal zueinander auf 0,001 mm genau bestimmt ($L_1$ und $L_2$). Anschließend wurden die Probekörper für 7 Tage bei 37 °C in Wasser gelagert. Nach 7 Tagen wurden die Probekörper herausgenommen, mit Wasser abgespült, abgetupft, für 15 Sekunden in der Luft hin- und hergeschwenkt und 1 Minute nach dem Herausnehmen aus dem Wasser wurde der Innendurchmesser an der Zylinderbasis an den gleichen zwei Stellen wie zuvor auf 0,001 mm genau bestimmt ($L_3$ und $L_4$). Nach der Messung wurden die Probekörper für weitere 7 Tage bei 37 °C in Wasser gelagert. Danach wurden die Probekörper herausgenommen, wie oben beschrieben getrocknet und erneut der Innendurchmesser an den gleichen Stellen auf 0,001 mm genau bestimmt ($L_5$ und $L_6$). Nach der Messung wurden die Probekörper für weitere 14 Tage bei 37 °C in Wasser gelagert. Danach wurden die Probekörper herausgenommen, wie oben beschrieben getrocknet und erneut der Innendurchmesser an den gleichen Stellen auf 0,001 mm genau bestimmt ($L_7$ und $L_8$). Nach der Messung wurden die Probekörper für weitere 28 Tage bei 37 °C in Wasser gelagert. Danach wurden die Probekörper herausgenommen, wie oben beschrieben getrocknet und erneut der Innendurchmesser an den gleichen Stellen auf 0,001 mm genau bestimmt ($L_9$ und $L_{10}$). Die lineare Quellung in % zu den jeweiligen Messterminen ergibt sich entsprechend der folgenden Formeln.

$$LQ_{1Woche}\,[\%] = \frac{\frac{L_3 + L_4}{2} - \frac{L_1 + L_2}{2}}{\frac{L_1 + L_2}{2}} \times 100\%$$

$$LQ_{2Wochen}\ [\%] = \frac{\frac{L_5 + L_6}{2} - \frac{L_1 + L_2}{2}}{\frac{L_1 + L_2}{2}} \times 100\%$$

$$LQ_{4Wochen}\ [\%] = \frac{\frac{L_7 + L_8}{2} - \frac{L_1 + L_2}{2}}{\frac{L_1 + L_2}{2}} \times 100\%$$

$$LQ_{8Wochen}\ [\%] = \frac{\frac{L_9 + L_{10}}{2} - \frac{L_1 + L_2}{2}}{\frac{L_1 + L_2}{2}} \times 100\%$$

Glührückstand: Zur Bestimmung des Glührückstandes wurden Tiegel für 10 Stunden auf 150 °C erwärmt, im Exsikkator auf Raumtemperatur abkühlen gelassen und anschließend auf 0,1 mg genau gewogen ($m_1$). Es wurden ca. 1 g des jeweiligen Komposit-Blockes zerkleinert, gemörsert und auf 0,1 mg genau im Tiegel eingewogen ($m_2$). Es wurde für 3 Stunden im Muffelofen auf 575 °C erhitzt, danach wurden die Tiegel im Exsikkator auf Raumtemperatur abkühlen gelassen und anschließend wurde die Masse auf 0,1 mg genau bestimmt ($m_3$). Der Glührückstand wurde nach folgender Formel berechnet.

$$Glührückstand\ [\%] = \frac{m_3 - m_1}{m_2} \times 100\%$$

Tabelle 1:

|  | Lava Ultimate (3M Espe) | Cerasmart (GC) | Block HC (Shofu) | Crios (Coltene) |
|---|---|---|---|---|
| Füllstoffgehalt (Herstellerangabe) [%] | 80 |  |  | 70.7 |
| Glührückstand [%] | 73 | 65 | 62 | 70 |
| Biaxiale Biegefestigkeit [MPa] | 174 | 214 | 147 | 232 |
| 3-Punkt-Biegefestigkeit [MPa] | 163 | 159 | 122 | 198 |
| E-Modul [GPa] | 11.8 | 9.9 | 8.7 | 12.7 |
| WSP [$\mu$g/mm$^3$] | 36 | 29 | 40 | 23 |
| WSP/E [$\mu$g/(GPa x mm$^3$)] | 3.05 | 2.93 | 4.60 | 1.81 |
| LQ (1 Woche) [%] | 0.23% | 0.19% | 0.27% | 0.15% |
| LQ (2 Wochen) [%] | 0.42% | 0.36% | 0.48% | 0.27% |
| LQ (4 Wochen) [%] | 0.51% | 0.42% | 0.60% | 0.34% |
| LQ (8 Wochen) [%] | 0.53% | 0.44% | 0.63% | 0.35% |

Tabelle 2:

| Beispiel | | | 1 | 2 | 3 | 4 |
|---|---|---|---|---|---|---|
| Füllstoff (a) | (a1a) | Dentalglas 1 | 13.00 | 13.00 | 13.00 | 13.00 |
| | (a1b) | Dentalglas 2 | 57.50 | 57.50 | 57.50 | 57.50 |
| | | Dentalglas 3 | | | | |
| | | Dentalglas 4 | | | | |
| | | Dentalglas 5 | | | | |
| | (a2) | Nano-SiO$_2$ (40 nm) | 15.00 | 15.00 | 15.00 | 15.00 |
| | Gesamt-(a) | | 85.50 | 85.50 | 85.50 | 85.50 |
| Monomere (b) | (b1a) | Bis-EMA2,6 | 6.00 | 6.00 | 6.00 | 6.50 |
| | | Bis-EMA4 | | | | |
| | (b1b) | Bis-EMA6 | | | | |
| | | Bis-EMA 10 | | | | |
| | (b2) | TCDDMA | 3.75 | 5.00 | 2.50 | 3.50 |
| | | UDMA | 3.75 | 2.50 | 5.00 | 3.50 |
| | | HDDMA | 0.70 | 0.70 | 0.70 | 0.70 |
| | | DODMA | | | | |
| | | TEGDMA | | | | |
| | Gesamt-(b) | | 14.20 | 14.20 | 14.20 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 3:

| Beispiel | 1 | 2 | 3 | 4 |
|---|---|---|---|---|
| (a1a)/(a1b) | 0.23 | 0.23 | 0.23 | 0.23 |
| (a2)/[(a1a)+(a1b)] | 0.21 | 0.21 | 0.21 | 0.21 |
| (b1a)/(b)×100% | 42.3% | 42.3% | 42.3% | 45.8% |
| Biaxiale Biegefestigkeit [MPa] | 301 | 269 | 292 | 284 |
| 3-Punkt-Biegefestigkeit [MPa] | 274 | 241 | 266 | 259 |
| E-Modul [GPa] | 18.3 | 15.8 | 18.6 | 16.4 |
| WSP [$\mu$g/mm$^3$] | 13 | 11 | 15 | 12 |
| WSP/E [$\mu$g/(GPa × mm$^3$)] | 0.71 | 0.70 | 0.81 | 0.73 |
| LQ (1 Woche) [%] | 0.09% | 0.09% | 0.10% | 0.08% |
| LQ (2 Wochen) [%] | 0.15% | 0.14% | 0.16% | 0.13% |
| LQ (4 Wochen) [%] | 0.19% | 0.18% | 0.19% | 0.17% |
| LQ (8 Wochen) [%] | 0.19% | 0.19% | 0.20% | 0.18% |

Tabelle 4:

| Beispiel | | | 5 | 6 | 7 | 8 |
|---|---|---|---|---|---|---|
| Füllstoff (a) | (a1a) | Dentalglas 1 | 13.00 | 13.00 | 13.00 | 13.00 |
| | (a1b) | Dentalglas 2 | 57.50 | 57.50 | 57.50 | 57.50 |
| | | Dentalglas 3 | | | | |
| | | Dentalglas 4 | | | | |
| | | Dentalglas 5 | | | | |
| | (a2) | Nano-SiO$_2$ (40 nm) | 15.00 | 15.00 | 15.00 | 15.00 |
| | Gesamt-(a) | | 85.50 | 85.50 | 85.50 | 85.50 |
| Monomere (b) | (b1a) | Bis-EMA2,6 | 7.00 | 5.80 | 6.00 | 6.00 |
| | | Bis-EMA4 | | | | |
| | (b1b) | Bis-EMA6 | | | | |
| | | Bis-EMA 10 | | | | |
| | (b2) | TCDDMA | 3.25 | 3.85 | 3.75 | 3.75 |
| | | UDMA | 3.25 | 3.85 | 3.75 | 3.75 |
| | | HDDMA | 0.70 | 0.70 | | |
| | | DODMA | | | 0.70 | |
| | | TEGDMA | | | | 0.70 |
| | Gesamt-(b) | | 14.20 | 14.20 | 14.20 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 5:

| Beispiel | 5 | 6 | 7 | 8 |
|---|---|---|---|---|
| (a1a)/(a1b) | 0.23 | 0.23 | 0.23 | 0.23 |
| (a2)/[(a1a)+(a1b)] | 0.21 | 0.21 | 0.21 | 0.21 |
| (b1a)/(b)x100% | 49.3% | 40.8% | 42.3% | 42.3% |
| Biaxiale Biegefestigkeit [MPa] | 271 | 295 | 261 | 299 |
| 3-Punkt-Biegefestigkeit [MPa] | 251 | 270 | 237 | 271 |
| E-Modul [GPa] | 15.1 | 18.7 | 15.1 | 19.1 |
| WSP [$\mu$g/mm$^3$] | 13 | 15 | 11 | 14 |
| WSP/E [$\mu$g/(GPa x mm$^3$)] | 0.86 | 0.80 | 0.73 | 0.73 |
| LQ (1 Woche) [%] | 0.09% | 0.09% | 0.05% | 0.07% |
| LQ (2 Wochen) [%] | 0.18% | 0.17% | 0.11% | 0.13% |
| LQ (4 Wochen) [%] | 0.21% | 0.20% | 0.17% | 0.16% |
| LQ (8 Wochen) [%] | 0.22% | 0.21% | 0.19% | 0.17% |

Tabelle 6:

| Beispiel | | | | 9 (Vergleich) | 10 | 11 (Vergleich) | 12 (Vergleich) |
|---|---|---|---|---|---|---|---|
| Füllstoff (a) | (a1a) | | Dentalglas 1 | | 13.00 | 13.00 | 13.00 |
| | (a1b) | | Dentalglas 2 | 70.50 | 57.50 | 57.50 | 57.50 |
| | | | Dentalglas 3 | | | | |
| | | | Dentalglas 4 | | | | |
| | | | Dentalglas 5 | | | | |
| | (a2) | | Nano-$SiO_2$ (40 nm) | 15.00 | 15.00 | 15.00 | 15.00 |
| | Gesamt-(a) | | | 85.50 | 85.50 | 85.50 | 85.50 |
| Monomere (b) | (b1a) | | Bis-EMA2,6 | 6.00 | | | |
| | | | Bis-EMA4 | | 6.00 | | |
| | (b1b) | | Bis-EMA6 | | | 6.00 | |
| | | | Bis-EMA10 | | | | 6.00 |
| | (b2) | | TCDDMA | 3.75 | 3.75 | 3.75 | 3.75 |
| | | | UDMA | 3.75 | 3.75 | 3.75 | 3.75 |
| | | | HDDMA | | 0.70 | 0.70 | 0.70 |
| | | | DODMA | | | | |
| | | | TEGDMA | | | | |
| | Gesamt-(b) | | | 14.20 | 14.20 | 14.20 | 14.20 |
| Initiatoren (c) | | | BPO | 0.30 | 0.30 | 0.30 | 0.30 |
| Gesamt | | | | 100.00 | 100.00 | 100.00 | 100.00 |

Tabelle 7:

| Beispiel | 9 (Vergleich) | 10 | 11 (Vergleich) | 12 (Vergleich) |
|---|---|---|---|---|
| (a1a)/(a1b) | 0.00 | 0.23 | 0.23 | 0.23 |
| (a2)/[(a1a)+(a1b)] | 0.21 | 0.21 | 0.21 | 0.21 |
| (b1a)/(b)x100% | 42.3% | 42.3% | 0.0% | 0.0% |
| Biaxiale Biegefestigkeit [MPa] | 256 | 289 | 262 | 219 |
| 3-Punkt-Biegefestigkeit [MPa] | 221 | 262 | 239 | 198 |
| E-Modul [GPa] | 13.4 | 17.7 | 13.7 | 12.6 |
| WSP [$\mu$g/mm$^3$] | 18 | 15 | 19 | 26 |
| WSP/E [$\mu$g/(GPa x mm$^3$)] | 1.34 | 0.85 | 1.39 | 2.06 |
| LQ (1 Woche) [%] | 0.13% | 0.10% | 0.15% | 0.17% |
| LQ (2 Wochen) [%] | 0.22% | 0.16% | 0.27% | 0.29% |
| LQ (4 Wochen) [%] | 0.28% | 0.21% | 0.31% | 0.36% |
| LQ (8 Wochen) [%] | 0.28% | 0.22% | 0.32% | 0.38% |

Tabelle 8:

| Beispiel | | | 13 | 14 (Vergleich) | 15 (Vergleich) | 16 (Vergleich) |
|---|---|---|---|---|---|---|
| Füllstoff (a) | (a1a) | Dentalglas 1 | 12.70 | 12.40 | 13.00 | 12.40 |
| | (a1b) | Dentalglas 2 | 56.10 | 54.70 | 57.50 | 54.70 |
| | | Dentalglas 3 | | | | |
| | | Dentalglas 4 | | | | |
| | | Dentalglas 5 | | | | |
| | (a2) | Nano-SiO$_2$ (40 nm) | 14.60 | 14.20 | 15.00 | 14.20 |
| | Gesamt-(a) | | 83.40 | 81.30 | 85.50 | 81.30 |
| Monomere (b) | (b1a) | Bis-EMA2,6 | 6.90 | 7.80 | 5.00 | 6.40 |
| | | Bis-EMA4 | | | | |
| | (b1b) | Bis-EMA6 | | | | |
| | | Bis-EMA10 | | | | |
| | (b2) | TCDDMA | 4.30 | 4.85 | 3.75 | 6.00 |
| | | UDMA | 4.30 | 4.85 | 3.75 | 6.00 |
| | | HDDMA | 0.80 | 0.90 | 1.70 | |
| | | DODMA | | | | |
| | | TEGDMA | | | | |
| | Gesamt-(b) | | 16.30 | 18.40 | 14.20 | 18.40 |
| Initiatoren (c) | | BPO | 0.30 | 0.30 | 0.30 | 0.30 |
| Gesamt | | | 100.00 | 100.00 | 100.00 | 100.00 |

Tabelle 9:

| Beispiel | 13 | 14 (Vergleich) | 15 (Vergleich) | 16 (Vergleich) |
|---|---|---|---|---|
| (a1a)/(a1b) | 0.23 | 0.23 | 0.23 | 0.23 |
| (a2)/[(a1a)+(a1b)] | 0.21 | 0.21 | 0.21 | 0.21 |
| (b1a)/(b)x100% | 42.3% | 42.4% | 35.2% | 34.8% |
| Biaxiale Biegefestigkeit [MPa] | 266 | 201 | 189 | 203 |
| 3-Punkt-Biegefestigkeit [MPa] | 240 | 175 | 169 | 177 |
| E-Modul [GPa] | 16.7 | 13.7 | 11.8 | 12.1 |
| WSP [$\mu$g/mm$^3$] | 15 | 19 | 16 | 17 |
| WSP/E [$\mu$g/(GPa x mm$^3$)] | 0.90 | 1.39 | 1.36 | 1.40 |
| LQ (1 Woche) [%] | 0.10% | 0.16% | 0.14% | 0.14% |
| LQ (2 Wochen) [%] | 0.17% | 0.26% | 0.24% | 0.26% |
| LQ (4 Wochen) [%] | 0.23% | 0.31% | 0.32% | 0.31% |
| LQ (8 Wochen) [%] | 0.23% | 0.31% | 0.32% | 0.32% |

Tabelle 10:

| Beispiel | | | 17 (Vergleich) | 18 (Vergleich) | 19 | 20 (Vergleich) |
|---|---|---|---|---|---|---|
| Füllstoff (a) | (a1a) | Dentalglas 1 | 13.00 | 12.40 | 12.70 | 12.70 |
| | (a1b) | Dentalglas 2 | 57.50 | | | |
| | | Dentalglas 3 | | 54.70 | | |
| | | Dentalglas 4 | | | 56.10 | |
| | | Dentalglas 5 | | | | 56.10 |
| | (a2) | Nano-SiO$_2$ (40 nm) | 15.00 | 14.20 | 14.60 | 14.60 |
| | Gesamt-(a) | | 85.50 | 81.30 | 83.40 | 83.40 |
| Monomere (b) | (b1a) | Bis-EMA2,6 | 7.50 | 7.80 | 6.90 | 6.90 |
| | | Bis-EMA4 | | | | |
| | (b1b) | Bis-EMA6 | | | | |
| | | Bis-EMA10 | | | | |
| | (b2) | TCDDMA | 3.00 | 4.85 | 4.30 | 4.30 |
| | | UDMA | 3.00 | 4.85 | 4.30 | 4.30 |
| | | HDDMA | 0.70 | 0.90 | 0.80 | 0.80 |
| | | DODMA | | | | |
| | | TEGDMA | | | | |
| | Gesamt-(b) | | 14.20 | 18.40 | 16.30 | 16.30 |
| Initiatoren (c) | | BPO | 0.30 | 0.30 | 0.30 | 0.30 |
| Gesamt | | | 100.00 | 100.00 | 100.00 | 100.00 |

Tabelle 11:

| Beispiel | 17 (Vergleich) | 18 (Vergleich) | 19 | 20 (Vergleich) |
|---|---|---|---|---|
| (a1a)/(a1b) | 0.23 | 0.23 | 0.23 | 0.23 |
| (a2)/[(a1a)+(a1b)] | 0.21 | 0.21 | 0.21 | 0.21 |
| (b1a)/(b)x100% | 52.8% | 42.4% | 42.3% | 42.3% |
| Biaxiale Biegefestigkeit [MPa] | 198 | 213 | 265 | 231 |
| 3-Punkt-Biegefestigkeit [MPa] | 177 | 194 | 242 | 211 |
| E-Modul [GPa] | 11.0 | 14.4 | 14.7 | 12.5 |
| WSP [$\mu$g/mm$^3$] | 15 | 20 | 16 | 17 |
| WSP/E [$\mu$g/(GPa x mm$^3$)] | 1.36 | 1.39 | 1.09 | 1.36 |
| LQ (1 Woche) [%] | 0.16% | 0.18% | 0.12% | 0.14% |
| LQ (2 Wochen) [%] | 0.25% | 0.27% | 0.19% | 0.23% |
| LQ (4 Wochen) [%] | 0.30% | 0.31% | 0.25% | 0.30% |
| LQ (8 Wochen) [%] | 0.31% | 0.31% | 0.26% | 0.31% |

Tabelle 12:

| Beispiel | | | 21 (Vergleich) | 22 (Vergleich) | 23 (Vergleich) | 24 (Vergleich) |
|---|---|---|---|---|---|---|
| Füllstoff (a) | (a1a) | Dentalglas 1 | 35.25 | 7.00 | 10.00 | 13.92 |
| | (a1b) | Dentalglas 2 | 35.25 | 60.10 | 44.20 | 61.58 |
| | | Dentalglas 3 | | | | |
| | | Dentalglas 4 | | | | |
| | | Dentalglas 5 | | | | |
| | (a2) | Nano-SiO$_2$ (40 nm) | 15.00 | 14.20 | 25.00 | 10.00 |
| | Gesamt-(a) | | 85.50 | 81.30 | 79.20 | 85.50 |
| Monomere (b) | (b1a) | Bis-EMA2,6 | 6.00 | 7.80 | 8.70 | 6.00 |
| | | Bis-EMA4 | | | | |
| | (b1b) | Bis-EMA6 | | | | |
| | | Bis-EMA10 | | | | |
| | (b2) | TCDDMA | 3.75 | 4.85 | 5.40 | 3.75 |
| | | UDMA | 3.75 | 4.85 | 5.40 | 3.75 |
| | | HDDMA | 0.70 | 0.90 | 1.00 | 0.70 |
| | | DODMA | | | | |
| | | TEGDMA | | | | |
| | Gesamt-(b) | | 14.20 | 18.40 | 20.50 | 14.20 |
| Initiatoren (c) | | BPO | 0.30 | 0.30 | 0.30 | 0.30 |
| Gesamt | | | 100.00 | 100.00 | 100.00 | 100.00 |

Tabelle 13:

| Beispiel | 21 (Vergleich) | 22 (Vergleich) | 23 (Vergleich) | 24 (Vergleich) |
|---|---|---|---|---|
| (a1a)/(a1b) | 1.00 | 0.12 | 0.23 | 0.23 |
| (a2)/[(a1a)+(a1b)] | 0.21 | 0.21 | 0.46 | 0.13 |
| (b1a)/(b)x100% | 42.3% | 42.4% | 42.4% | 42.3% |
| Biaxiale Biegefestigkeit [MPa] | 204 | 197 | 156 | 188 |
| 3-Punkt-Biegefestigkeit [MPa] | 181 | 174 | 128 | 145 |
| E-Modul [GPa] | 11.7 | 14.3 | 11.7 | 12.4 |
| WSP [$\mu$g/mm$^3$] | 19 | 21 | 31 | 21 |
| WSP/E [$\mu$g/(GPa x mm$^3$)] | 1.62 | 1.47 | 2.65 | 1.69 |
| LQ (1 Woche) [%] | 0.19% | 0.20% | 0.23% | 0.21% |
| LQ (2 Wochen) [%] | 0.27% | 0.26% | 0.37% | 0.29% |
| LQ (4 Wochen) [%] | 0.32% | 0.31% | 0.43% | 0.34% |
| LQ (8 Wochen) [%] | 0.33% | 0.32% | 0.45% | 0.35% |

**Patentansprüche**

**1.** Dentaler Fräsrohling zur Herstellung permanenter indirekter Restaurationen im CAD/CAM Verfahren, **dadurch**

**gekennzeichnet, dass** er eine Wasseraufnahme WSP von kleiner/gleich 15 $\mu$g/mm$^3$, gemessen nach ISO 4049 und einen E-Modul E größer/gleich 15 GPa, gemessen nach der ADA Spezifikation No. 27 und einen Quotient Q aus WSP/E von kleiner 1 $\mu$g/(GPa x mm$^3$) aufweist und aus dem Polymerisat einer radikalisch härtbaren dentalen Zusammensetzung besteht, die umfasst

a) anorganische Füllstoffe, wobei die Gesamtmasse der anorganischen Füllstoffe mindestens 83 Gew.-%, bezogen auf die Gesamtmasse der Zusammensetzung, beträgt,
b) radikalisch polymerisierbare Monomere,
c) einen oder mehrere Initiatoren zur radikalischen Härtung,

wobei die anorganischen Füllstoffe a) umfassen
a1) eine Glaszusammensetzung und
a2) nicht aggregierte und nicht agglomerierte Kieselsäure mit einer mittleren Teilchengröße von nicht mehr als 80 nm,
wobei die Glaszusammensetzung a1) eine erste Glaszusammensetzung a1a) mit einem D50-Wert von 0,4 - 1,0 $\mu$m und eine zweite Glaszusammensetzung a1b) mit einem D50-Wert von 1,2 - 5,0 $\mu$m umfasst und wobei das Massenverhältnis von a1a) zu a1b) zwischen 1 : 1,5 und 1 : 8 und das Massenverhältnis von a2) zur Summe von a1a) und a1b) zwischen 1 3 und 1 6 liegt und das Verhältnis der mittleren Korngröße der ersten Mikropartikelfraktion (a1a) zur mittleren Korngröße der zweiten Mikropartikelfraktion (a1b) im Bereich von 1 : 1,5 bis 1 : 10 liegt,
wobei der D75-Wert von a1a) kleiner ist als der D25-Wert von a1b).

2.  Dentaler Fräsrohling zur Herstellung permanenter indirekter Restaurationen im CAD/CAM Verfahren nach Anspruch 1, wobei die radikalisch polymerisierbaren Monomeren b) difunktionelle (Meth)acrylate umfassen und wobei der Gewichtsanteil von ethoxyliertem Bisphenol-A-Dimethacrylat mit einem durchschnittlichen Ethoxylierungsgrad von 2 bis 4 Ethoxygruppen pro Molekül größer 40% Gew.-% und kleiner 50 Gew.-% an b) ist.

3.  Dentaler Fräsrohling zur Herstellung permanenter indirekter Restaurationen im CAD/CAM Verfahren nach Anspruch 1 oder 2, wobei die Glaszusammensetzung a1) eine erste Glaszusammensetzung a1a) mit einem D50-Wert von 0,5 - 0,9 $\mu$m und eine zweite Glaszusammensetzung a1b) mit einem D50-Wert von 1,5 - 4,0 $\mu$m umfasst und wobei das Massenverhältnis von a1a) zu a1b) zwischen 1 : 2 bis 1 : 5 und das Massenverhältnis von a2) zur Summe von a1a) und a1b) zwischen 1 : 3 und 1 : 6 liegt und das Verhältnis der mittleren Korngröße der ersten Mikropartikelfraktion (a1a) zur mittleren Korngröße der zweiten Mikropartikelfraktion (a1b) im Bereich von 1 : 2 bis 1 : 5 liegt, wobei der D75-Wert von a1a) kleiner ist als der D25-Wert von a1b).

4.  Dentaler Fräsrohling zur Herstellung permanenter indirekter Restaurationen im CAD/CAM Verfahren nach einem der Ansprüche 1 bis 3, wobei die anorganischen Füllstoffe a) organisch oberflächenbeschichtet, vorzugsweise silanisiert und am meisten bevorzugt mit Methacryloxypropyltrimethoxysilan behandelt sind.

5.  Dentaler Fräsrohling zur Herstellung permanenter indirekter Restaurationen im CAD/CAM Verfahren nach Anspruch 1 bis 4, wobei der Anteil der nicht aggregierten und nicht agglomerierten Kieselsäure mit einer mittleren Teilchengröße von nicht mehr als 80 nm größer als 11,86 Gew.-% und kleiner als 23 Gew.-%, bezogen auf die Gesamtzusammensetzung, ist.

6.  Dentaler Fräsrohling zur Herstellung permanenter indirekter Restaurationen im CAD/CAM Verfahren nach einem der Ansprüche 1 bis 5, wobei die Glaszusammensetzung a1) eine Barium-Aluminium-Borsilikatzusammensetzung ist.

7.  Dentaler Fräsrohling zur Herstellung permanenter indirekter Restaurationen im CAD/CAM Verfahren nach einem der vorangehenden Ansprüche, wobei die radikalisch härtbare Zusammensetzung zusätzlich Additive d) umfasst.

8.  Dentaler Fräsrohling zur Herstellung permanenter indirekter Restaurationen im CAD/CAM Verfahren nach einem der vorangehenden Ansprüche, wobei die Menge an radikalisch polymerisierbaren Monomeren b) in einem Bereich von höchstens 16,7 Gew.-%, bezogen auf die Gesamtzusammensetzung, liegt und die Menge an einem oder mehreren Initiator(en) zur radikalischen Härtung c) in einem Bereich von 0,2 bis 5 Gew.-%, bezogen auf die Gesamtzusammensetzung, sowie die Menge des Additivs oder der Additive d) in einem Bereich von 0,001 Gew.-% bis 2 Gew.-%, bezogen auf die Gesamtzusammensetzung, liegt.

9. Verfahren zur Herstellung eines dentalen Fräsrohlings nach einem der vorangehenden Ansprüche mit den folgenden Schritten:

- Bereitstellen der Bestandteile a), b), c) und gegebenenfalls d),
- homogenes Mischen der Bestandteile und
- Härten der Bestandteile.

10. Kit, umfassend

- mehrere dentale Fräsrohlinge nach einem der Ansprüche 7 oder 8 in unterschiedlichen Farben,
- wenigstens einen Primer,
- wenigstens ein dentales Adhäsiv,
- wenigstens einen Befestigungszement sowie
- optional weiteres Zubehör wie Pinsel, Poliermittel und Mischkanülen.

**Claims**

1. Dental milling blank for the production of permanent indirect restorations in the CAD/CAM process, **characterized in that** it has a water sorption WSP of less than/equal to 15 $\mu g/mm^3$, measured according to ISO 4049 and an E modulus E greater than/equal to 15 GPa, measured according to the ADA specification No. 27 and a quotient Q of WSP/E of less than 1 $\mu g/(GPa \times mm^3)$ and consists of the polymerization product of a radically curable dental composition, which comprises

a) inorganic fillers, wherein the total mass of the inorganic fillers is at least 83 wt.%, based on the total mass of the composition,
b) radically polymerizable monomers,
c) one or more initiators for radically curing,

wherein the inorganic fillers a) comprise
a1) a glass composition and
a2) non-aggregated and non-agglomerated silicic acid with an average particle size of not more than 80 nm, wherein the glass composition a1) comprises a first glass composition a1a) with a D50 value from 0.4 - 1.0 $\mu m$ and a second glass composition a1b) with a D50 value from 1.2 - 5.0 $\mu m$ and wherein the mass ratio of a1a) to a1b) lies between 1:1.5 and 1:8 and the mass ratio of a2) to the sum of a1a) and a1b) lies between 1:3 and 1:6 and the ratio of the average particle size of the first microparticle fraction (a1a) to the average particle size of the second microparticle fraction (a1b) lies in the range from 1:1.5 to 1:10, wherein the D75 value of a1a) is smaller than the D25 value of a1b).

2. Dental milling blank for the production of permanent indirect restorations in the CAD/CAM process according to claim 1, wherein the radically polymerizable monomers b) comprise bifunctional (meth)acrylates and wherein the proportion by weight of ethoxylated bisphenol-A dimethacrylate with an average degree of ethoxylation of 2 to 4 ethoxy groups per molecule is greater than 40 wt.% and less than 50 wt.% of b).

3. Dental milling blank for the production of permanent indirect restorations in the CAD/CAM process according to claim 1 or 2, wherein the glass composition a1) comprises a first glass composition a1a) with a D50 value from 0.5 - 0.9 $\mu m$ and a second glass composition a1b) with a D50 value from 1.5 - 4.0 $\mu m$ and wherein the mass ratio of a1a) to a1b) lies between 1:2 to 1:5 and the mass ratio of a2) to the sum of a1a) and a1b) lies between 1:3 and 1:6 and the ratio of the average particle size of the first microparticle fraction (a1a) to the average particle size of the second microparticle fraction (a1b) lies in the range from 1:2 to 1:5, wherein the D75 value of a1a) is smaller than the D25 value of a1b).

4. Dental milling blank for the production of permanent indirect restorations in the CAD/CAM process according to one of the claims 1 to 3, wherein the inorganic fillers a) are organically surface-coated, preferably silanized and most preferably treated with methacryloxypropyltrimethoxysilane.

5. Dental milling blank for the production of permanent indirect restorations in the CAD/CAM process according to claim 1 to 4, wherein the content of the non-aggregated and non-agglomerated silicic acid with an average particle size of not more than 80 nm is greater than 11.86 wt.% and less than 23 wt.%, based on the total composition.

6. Dental milling blank for the production of permanent indirect restorations in the CAD/CAM process according to one of the claims 1 to 5, wherein the glass composition a1) is a barium-aluminum borosilicate composition.

7. Dental milling blank for the production of permanent indirect restorations in the CAD/CAM process according to one of the preceding claims, wherein the radically curable composition additionally comprises additives d).

8. Dental milling blank for the production of permanent indirect restorations in the CAD/CAM process according to one of the preceding claims, wherein the quantity of radically polymerizable monomers b) lies in a range of at most 16.7 wt.%, based on the total composition, and the quantity of one or more initiator(s) for the radically curing c) lies in a range from 0.2 to 5 wt.%, based on the total composition, and the quantity of the additive or additives d) lies in a range from 0.001 wt.% to 2 wt.%, based on the total composition.

9. A process for the production of a dental milling blank according to one of the preceding claims with the following steps:

- providing the constituents a), b), c) and optionally d),
- homogeneously mixing the constituents and
- curing the constituents.

10. A kit, comprising

- several dental milling blanks according to one of the claims 7 or 8 in different colors,
- at least one primer,
- at least one dental adhesive,
- at least one luting cement and
- optionally further accessories such as brushes, polishing agents and mixing tips.

**Revendications**

1. Ébauche de fraisage dentaire pour la fabrication de restaurations indirectes permanentes dans un procédé CAD/CAM, **caractérisée en ce qu'**elle présente une absorption d'eau WSP inférieure ou égale à 15 $\mu$g/mm$^3$, mesurée selon la norme ISO 4049 et un module d'élasticité E supérieur ou égal à 15 GPa, mesuré selon la spécification ADA n° 27 et un quotient Q WSP/E inférieur à 1 $\mu$g/(GPa x mm$^3$) et est constitué d'un polymère d'une composition dentaire durcissable par voie radicalaire, qui comprend

a) des charges inorganiques, la masse totale des charges inorganiques étant d'au moins 83 % en poids, par rapport à la masse totale de la composition,
b) des monomères polymérisables par voie radicalaire,
c) un ou plusieurs amorceurs de durcissement radicalaire,

les charges inorganiques a) comprenant
a1) une composition de verre et
a2) de la silice non agrégée et non agglomérée ayant une granulométrie moyenne non supérieure à 80 nm, la composition de verre a1) comprenant une première composition de verre a1a) ayant une D50 de 0,4 à 1,0 $\mu$m et une seconde composition de verre a1b) ayant une D50 de 1,2 à 5,0 $\mu$m, et le rapport en masse de a1a) à a1b) étant compris entre 1:1,5 et 1:8, et le rapport en masse de a2) à la somme de a1a) et de a1b) étant compris entre 1:3 et 1:6, et le rapport entre la granulométrie moyenne de la première fraction de microparticules (a1a) et la granulométrie moyenne de la seconde fraction de microparticules (a1b) étant compris dans la plage de 1:1,5 à 1:10, la D75 de a1a) étant inférieure à la D25 de a1b).

2. Ébauche de fraisage dentaire pour la fabrication de restaurations indirectes permanentes dans un procédé CAD/CAM selon la revendication 1, les monomères b) polymérisables par voie radicalaire comprenant des (méth)acrylates difonctionnels, et la proportion en poids du diméthacrylate de bisphénol A éthoxylé ayant un degré d'éthoxylation moyen de 2 à 4 groupes éthoxy par molécule étant supérieure à 40 % en poids et inférieure à 50 % en poids de b).

**3.** Ébauche de fraisage dentaire pour la fabrication de restaurations indirectes permanentes dans un procédé CAD/CAM selon la revendication 1 ou 2, la composition de verre a1) comprenant une première composition de verre a1a) ayant une D50 de 0,5 à 0,9 $\mu$m et une seconde composition de verre a1b) ayant une D50 de 1,5 à 4,0 $\mu$m, et le rapport en masse de a1a) à a1b) étant compris entre 1:2 et 1:5, et le rapport en masse de a2) à la somme de a1a) et de a1b) étant compris entre 1:3 et 1:6, et le rapport entre la granulométrie moyenne de la première fraction de microparticules (a1a) et la granulométrie moyenne de la seconde fraction de microparticules (a1b) étant compris dans la plage de 1:2 à 1:5,
la D75 de a1a) étant inférieure à la D25 de a1b).

**4.** Ébauche de fraisage dentaire pour la fabrication de restaurations indirectes permanentes dans un procédé CAD/CAM selon l'une des revendications 1 à 3, les charges inorganiques a) étant revêtues en surface d'un revêtement organique, de préférence silanées, et tout spécialement étant traitées par du méthacryloxypropyltriméthoxysilane.

**5.** Ébauche de fraisage dentaire pour la fabrication de restaurations indirectes permanentes dans un procédé CAD/CAM selon les revendications 1 à 4, la proportion de la silice non agrégée et non agglomérée ayant une granulométrie non supérieure à 80 nm étant supérieure à 11,86 % en poids et inférieure à 23 % en poids par rapport à la composition totale.

**6.** Ébauche de fraisage dentaire pour la fabrication de restaurations indirectes permanentes dans un procédé CAD/CAM selon l'une des revendications 1 à 5, la composition de verre a1) étant une composition de silicate de baryum-aluminium-bore.

**7.** Ébauche de fraisage dentaire pour la fabrication de restaurations indirectes permanentes dans un procédé CAD/CAM selon l'une des revendications précédentes, la composition durcissable par voie radicalaire comprenant en outre des additifs d).

**8.** Ébauche de fraisage dentaire pour la fabrication de restaurations indirectes permanentes dans un procédé CAD/CAM selon l'une des revendications précédentes, la quantité des monomères b) polymérisables par voie radicalaire étant comprise dans une plage d'au plus 16,7 % en poids par rapport à la composition totale, et la quantité d'un ou plusieurs amorceurs de durcissement radicalaire c) étant comprise dans une plage de 0,2 à 5 % en poids, par rapport à la composition totale, et la quantité de l'additif ou des additifs d) étant comprise dans une plage de 0,001 % en poids à 2 % en poids par rapport à la composition totale.

**9.** Procédé de fabrication d'une ébauche de fraisage dentaire selon l'une des revendications précédentes, comportant les étapes suivantes :

- mise à disposition des constituants a), b), c) et éventuellement d),
- mélange homogène des constituants et
- durcissement des constituants.

**10.** Kit comprenant

- plusieurs ébauches de fraisage dentaire selon l'une des revendications 7 ou 8 en différentes couleurs,
- au moins un apprêt,
- au moins un adhésif dentaire,
- au moins un ciment de fixation ainsi que
- éventuellement d'autres accessoires tels qu'un pinceau, un agent de polissage et des aiguilles de transfert.

Abbildung 1

Abbildung 2

SECTION B-B

Abbildung 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 69922413 T2
- EP 3050533 A1
- DE 2656847
- DE 2462271 C2
- EP 2623065 B1
- WO 2011087832 A1
- WO 2016140950 A1
- EP 2881077 A1
- US 6345984 B2
- US 4544359 A
- US 6186790 B
- DE 19823530 B4
- DE 60111868 T2
- US 5962550 A
- US 4616073 A
- US 6030606 A
- DE 102015220373 A1
- US 20140200284 A1
- DE 1816823
- DE 2419887
- DE 2406557
- DE 2931926
- DE 3522005
- DE 3522006
- DE 3703120
- DE 102005021332
- DE 102005053775
- DE 102006060983
- DE 69935794
- DE 102007034457
- DE 102006019092 A1
- DE 3941629 C2
- DE 102006019092
- DE 60116142
- DE 3801511 C2

- DE 102006050153 A1
- EP 0184095 B1
- DE 4231579 C2
- EP 0366977 B1
- US 7081485 B2
- DE 3236026 A1
- US 20070027229 A1
- EP 0262629 B1
- EP 0073413 A
- US 7148382 B2
- US 5761169 A
- DE 19708294 A1
- EP 0057474 A
- EP 0047902 A
- EP 0007508 A
- DE 60029481 T2
- EP 0980682 B1
- EP 0948955 B1
- EP 1236459 B1
- EP 0173567 A2
- EP 1720506 A
- DE 3941629
- EP 1839640 A
- DE 1495520
- WO 02092021 A
- WO 02092023 A
- EP 1872767 A
- EP 2070506 A
- EP 1881010 A
- DE 102007050763
- US 6288138 B
- DE 112006001049
- US 7214726 B
- EP 2070935 A
- EP 0059451 A

### In der Beschreibung aufgeführte Nicht-Patentliteratur

- *Journal of Prosthodontic Research,* 2010, vol. 54, 59-64
- **J.-P. FOUASSIER.** Photoinitiation, Photopolymerization and Photocuring. Hanser Publishers, 1995

- Radiation Curing in Polymer Science and Technology. Elsevier Applied Science, 1993, vol. II